# EUROPÄISCHE PATENTSCHRIFT

(11) **EP 1 673 629 B1**
(45) Veröffentlichungstag und Bekanntmachung des Hinweises auf die Patenterteilung: **11.08.2010**
(21) Anmeldenummer: 04790371.1
(22) Anmeldetag: 13.10.2004
(51) Int. Cl.: G01N 33/50

(54) **IN VITRO VERFAHREN ZUR DIAGNOSE DER KARDIOVASKULÄREN FUNKTIONALITÄT VON KNOCHENMARKS-VORLÄUFERZELLEN (BMP) UND/ODER BLUT-ABGELEITETER ZIRKULIERENDER VORLÄUFERZELLEN (BDP)**
IN VITRO METHOD FOR THE DIAGNOSIS OF CARDIOVASCULAR FUNCTIONALITY OF BONE MARROW PRECURSOR CELLS (BMP) AND/OR CIRCULATION PRECURSOR CELLS DERIVED FROM BLOOD (BDP)
METHODE IN VITRO DE DIAGNOSTIC DE LA FONCTIONNALITE CARDIO-VASCULAIRE DE CELLULES PRECURSEURS DE MOELLE OSSEUSE (BMP) ET/OU DE CELLULES PRECURSEURS EN CIRCULATION, DERIVEES DU SANG

(30) Priorität: 13.10.2003 DE 10347436
(43) Veröffentlichungstag der Anmeldung: 28.06.2006
(73) Patentinhaber: t2cure GmbH, 60325 Frankfurt am Main (DE)
(72) Erfinder: ZEIHER, Andreas, M., 60594 Frankfurt (DE); HEESCHEN, Christopher, Dr., 81247 München (DE); DIMMELER, Stefanie, 60594 Frankfurt (DE)
(74) Vertreter: Krauss, Jan
(86) Internationale Anmeldenummer: PCT/EP2004/011503
(87) Internationale Veröffentlichungsnummer: WO 2005/038454

(56) Entgegenhaltungen:
- VASA M ET AL: "Number and migratory activity of circulating endothelial progenitor cells inversely correlate with risk factors for coronary artery disease." CIRCULATION RESEARCH. 6 JUL 2001, Bd. 89, Nr. 1, 6. Juli 2001 (2001-07-06), Seiten E1-E7, XP002313345 ISSN: 1524-4571 in der Anmeldung erwähnt
- YAMAGUCHI J-I ET AL: "Stromal cell-derived factor-1 effects on ex vivo expanded endothelial progenitor cell recruitment for ischemic neovascularization" CIRCULATION 11 MAR 2003 UNITED STATES, Bd. 107, Nr. 9, 11. März 2003 (2003-03-11), Seiten 1322-1328, XP002313346 ISSN: 0009-7322
- ASSMUS BIRGIT ET AL: "Transplantation of progenitor cells and regeneration enhancement in acute myocardial infarction (TOPCARE-AMI)." CIRCULATION, Bd. 106, Nr. 24, 10. Dezember 2002 (2002-12-10), Seiten 3009-3017, XP002313347 ISSN: 0009-7322 in der Anmeldung erwähnt
- KIM C H ET AL: "IN VITRO BEHAVIOR OF HEMATOPOIETIC PROGENITOR CELLS UNDER THE INFLUENCE OF CHEMOATTRACTANTS: STROMAL CELL-DERIVED FACTOR-1, STEEL FACTOR, AND THE BONE MARROW ENVIRONMENT" BLOOD, W.B. SAUNDERS, PHILADELPHIA, VA, US, Bd. 91, Nr. 1, 1998, Seiten 100-110, XP000866137 ISSN: 0006-4971

## Beschreibung

Die vorliegende Erfindung betrifft ein *in vitro* Verfahren zur Stratifizierung von Patienten mit kardiovaskulären Erkrankungen für eine geplante Zelltherapie auf Basis von Knochenmarks-Vorläuferzellen (BMP) und/oder Blut-abgeleiteter zirkulierender Vorläuferzellen (BDP) zur Steigerung der Perfusion von ischämischem Gewebe bzw. zur Regeneration von Gewebeverlust, wobei das Verfahren folgende Schritte umfasst: a) Isolieren von BMP und/oder BDP mittels zellspezifischer Oberflächenmarker aus einer Probe, und b) Überprüfung der kardiovaskulären Funktionalität der isolierten BMP und/oder BDP mittels eines geeigneten Migrationstests. Das Verfahren kann im Rahmen der Diagnose und/oder der Prognose kardiovaskulärer Erkrankungen, zur Überwachung von deren Therapie und/oder zur Stratifizierung für eine geplante Zelltherapie mit Stamm- und/oder Vorläuferzellen zur Steigerung der Perfusion von ischämischem Gewebe bzw. zur Regeneration von Gewebeverlust (z. B. Herzinsuffizienz) angewendet werden. In einem weiteren Aspekt beschreibt die vorliegende Offenbarung dann ein *in vitro* Verfahren zur Isolierung von spezifischen Knochenmarks-Vorläuferzellen (BMP) und/oder Blut-abgeleiteten zirkulierenden Vorläuferzellen (BDP), umfassend: a) Entnehmen einer Probe aus einem Säugetier-Spender, b) Isolieren von BMP und/oder BDP mittels zellspezifischer Oberflächenmarker aus der so erhaltenen Probe, und c) Überprüfung der kardiovaskulären Funktionalität der isolierten BMP und/oder BDP mittels eines geeigneten Migrationstests. Die kardiovaskulären Erkrankungen sind ausgewählt aus der Gruppe bestehend aus stabiler koronarer Herzerkrankung, akutem Koronarsyndrom, akutem Myokardinfarkt, chronischer ischämischer Kardiomyopathie (ICMP), dilatativer Kardiomyopathie (DCM) oder anderer Ursachen einer Herzschwäche.

Post-Infarkt Herzversagen bleibt eine Hauptursache von Morbidität und Mortalität. Obwohl eine prompte Reperfusion der verschlossenen Arterie signifikant verringerte Mortalitätsraten bewirkt (Lange RA, Hillis LD. Reperfusion therapy in acute myocardial infarction. N Engl J Med. 2002:346:954-5.), führen ventrikuläre Remodelling-Prozesse, die durch die progressive Expansion des Infarktbereichs und Dilatation der linken ventrikulären Kavität gekennzeichnet sind zu der Entwicklung von Herzversagen bei einem messbaren Teil der Patienten, die einen akuten myokardialen Infarkt überleben (Pfeffer MA, Braunwald E. Ventricular remodeling after myocardial infarction. Experimental observations and clinical implications. Circulation. 1990:81:1161 -72.). Das hauptsächliche Ziel, um ein Remodelling umzukehren, wäre die Stimulierung der Neovaskularisierung sowie die Verstärkung der Regeneration von kardialen Myocyten innerhalb des Infarktbereichs.

Kürzliche Experimente und klinische Studien haben gezeigt, dass die Transplantation von spezifischen Knochenmarks-Vorläuferzellen (BMP) und/oder Blut-abgeleiteten zirkulierender Vorläuferzellen (BDP) die Regeneration nach einem akuten Myokardinfarkt verbessert (Kawamoto A, Gwon HC, Iwaguro H, Yamaguchi Jl, Uchida S, Masuda H, Silver M, Ma H, Kearney M, Isner JM, Asahara T. Therapeutic potential of ex vivo expanded endothelial progenitor cells for myocardial ischemia. Circulation. 2001; 103:634-7., Kocher AA, Schuster MD, Szabolcs MJ, Takuma S, Burkhoff D, Wang J, Homma S, Edwards NM, Itescu S. Neovascularization of ischemic myocardium by human bone-marrow-derived angioblasts prevents cardiomyocyte apoptosis, reduces remodeling and improves cardiac function. Nat Med. 2001:7:430-6., Fuchs S, Baffour R, Zhou YF, Shou M, Pierre A, Tio FO, Weissman NJ, Leon MB, Epstein SE, Komowski R. Transendocardial delivery of autologous bone marrow enhances collateral perfusion and regional function in pigs with chronic experimental myocardial ischemia. J Am Coil Cardiol. 2001;37:1726-32.). Die Zelltherapie mit Knochenmarks-Stamm-/Vorläuferzellen ist daher eine neue Option zur Verbesserung der Neovaskularisierung und der kardialen Funktion bei ischämischer Herzerkrankung. So konnte in der Tat gezeigt werden, dass die intrakoronare Infusion von adulten Vorläuferzellen bei Patienten mit akutem Myokardialinfarkt durchführbar und sicher ist und mit signifikanten Verbesserungen in der regionalen und globalen LV (left ventricular) Funktion assoziiert ist. Die genauen Umstände der Verbesserung durch die Infusion blieben jedoch unklar. Für eine gezielte und effektive Zelltherapie mit Knochenmarks-Stamm-/Vorläuferzellen ist es zudem erforderlich, sicherzustellen, dass Knochenmarks-Vorläuferzellen (BMP) und/oder Blut-abgeleitete zirkulierende Vorläuferzellen (BDP) transplantiert werden, die eine möglichst hohe Regenerationsfähigkeit und somit Effektivität aufweisen.

Es ist somit eine Aufgabe der vorliegenden Erfindung, ein verbessertes Verfahren zur Stratifizierung von Patienten mit kardiovaskulären Erkrankungen für eine geplante Zelltherapie auf Basis von Knochenmarks-Vorläuferzellen (BMP) und/oder Blut-abgeleiteten zirkulierender Vorläuferzellen (BDP) zur Steigerung der Perfusion von ischämischem Gewebe bzw. zur Regeneration von Gewebeverlust bei z. B. Herzinsuffizienz zur Verfügung zu stellen.

Die vorliegende Offenbarung beschreibt außerdem ein sicheres Verfahren, mit dem effektive und für die Zelltherapie geeignete Knochenmarks-Vorläuferzellen (BMP) und/oder Blut-abgeleiteten zirkulierenden Vorläuferzellen (BDP) identifiziert und isoliert werden können.

Gemäß eines ersten Aspekts der vorliegenden Erfindung wird eine Aufgabe der Erfindung durch ein *in vitro* Verfahren zur Stratifizierung von Patienten mit kardiovaskulären Erkrankungen für eine geplante Zelltherapie auf Basis von Knochenmarks-Vorläuferzellen (BMP) und/oder Blut-abgeleiteter zirkulierender Vorläuferzellen (BDP) zur Steigerung der Perfusion von ischämischem Gewebe bzw. zur Regeneration von Gewebeverlust, wobei das erfindungsgemässe Verfahren folgende Schritte umfasst von: a) Isolieren von Knochenmarks-Vorläuferzellen (BMP) und/oder Blut-abgeleiteten zirkulierenden Vorläuferzellen (BDP) mittels zellspezifischer Oberflächenmarker, und b) Überprüfung/Ermittlung der kardiovaskulären Funktionalität der isolierten BMP und/oder BDP mittels eines geeigneten Migrationstests.

Überraschend konnte nun gefunden werden, dass ein direkter Zusammenhang zwischen der Neovaskularisierungskapazität und der Migrationsfähigkeit von Knochenmarks-Vorläuferzellen (BMP) und/oder Blut-abgeleiteten zirkulierenden Vorläuferzellen (BDP) besteht. Dies ermöglicht den Einsatz eines Migrationstests als wertvolles Werkzeug zur Analyse einer Probe aus einem Säugetier in Zusammenhang mit kardiovaskulären Erkrankungen.

Weiterhin konnte überraschend gefunden werden, dass Vorläuferzellen aus dem Knochenmark einiger Spender und/oder Herzinfarktpatienten eine Dysfunktion aufweisen, wodurch die Zellen, die man direkt aus dem Knochenmark entnehmen kann (BMP, zum Beispiel durch Punktion) in ihrer Funktion eingeschränkt sind, neue Gefäße bilden zu können und die Herzregeneration zu unterstützen. Durch den erfindungsgemäßen Test können solche Spender effektiv ausgeschlossen werden. Gleichzeitig können Patienten identifiziert werden, die aufgrund der geringen migratorischen Fähigkeit ihrer BMP oder BDP ein erhöhtes Risiko aufweisen. Diese Patienten können dann geeignet und insbesondere präventiv und/oder unterstützend behandelt werden. Alternativ können durch diesen Test auch Patienten identifiziert werden, die durch eine Vorläuferzell-Therapie einen erhöhten Nutzen erfahren können. Diese beiden oben genannten Eigenschaften der BMP und/oder BDP, die Migrationsfähigkeit und Neovaskularisierungskapazität der Zellen, werden im Rahmen der vorliegenden Erfindung als "kardiovaskuläre Funktionalität" verstanden.

Vasa et al. (Vasa M. et al., Circ Res 2001 Jul 6;89(1):E1-7) beschreiben, dass die Zahl und migratorische Aktivität von zirkulierenden Endothel-Vorläuferzellen (EPCs) invers mit Risikofaktoren für koronare Arterienkrankungen (CAD) korrelieren. EPCs von Patienten mit koronaren Arterienkrankungen zeigten funktionelle Beeinträchtigungen. Die funktionelle Aktivität der EPCs wird untersucht anhand der migratorischen Kapazität, die mit Hilfe eines Migrationstests in einer Boyden-Kammer bestimmt wird (Wanderung in Richtung VEGF als "chemoattractant"). Die postnatale Neovaskularisierung erfolgt unter Beteiligung Knochenmark-abgeleiteter zirkulierender Vorläuferzellen (EPCs), die von hämatopoietischen Stammzellen abstammen, die charakterisiert sind durch den Zelloberflächenmarker CD34 oder CD 133 für weniger ausgereifte Zellen. Die CD34⁺ Zellen siedeln sich in ischämischen Bereichen in Mausmodellen an, die Injektion von CD34⁺ Zellen fördert in den Versuchen die Neovaskularisierung und verbessert die Herzfunktion in diesen Mäusen. Weiter wird die Anzahl und funktionelle Aktivität von EPCs in Patienten mit CAD (coronary artery disease) im Vergleich zu gesunden Personen untersucht, auch der Einfluss von Risikofaktoren auf Anzahl und funktionelle Aktivität der EPCs. Die Anzahl CD34⁺ KDR⁺ (=VEGF Rezeptor) doppelt positiver Zellen ist deutlich niedriger in Patienten mit CAD als in gesunden Personen, jedoch ist die Gesamtanzahl von CD34⁺ Zellen oder weniger differenzierter CD133⁺ Zellen nicht unterschiedlich in Patienten und gesunden Personen. Die migratorische Kapazität der EPCs ist reduziert in Patienten mit CAD. Vasa et al. äussem die Vermutung, dass die reduzierte Aktivität der EPCs zu einer verringerten Neovaskularisierung in Patienten mit CAD beitragen könnte. Die Möglichkeit einer Stratifizierung von Patienten auf Basis einer Analyse von Knochenmarks-Vorläuferzellen (BMP) und/oder Blut-abgeleiteter zirkulierender Vorläuferzellen (BDP) wird jedoch nicht erwähnt.

Weiterhin beschreiben Vasa et al. (Vasa M. et al., Circulation, 2001 Jun 19;103(24):2885-90) eine Zunahme von zirkulierenden Endothel-Vorläuferzellen (EPCs) durch Statin-Therapie in Patienten mit stabiler CAD. Dabei wird die Neovaskularisierung durch EPCs durch Statine unterstützt. Die funktionelle Aktivität der EPCs wird durch einen migratorischen Test bestimmt. Die Möglichkeit einer Stratifizierung von Patienten auf Basis einer Analyse von Knochenmarks-Vorläuferzellen (BMP) und/oder Blut-abgeleiteten zirkulierenden Vorläuferzellen (BDP) wird jedoch ebenfalls nicht erwähnt.

Emerson et al. (in Circulation 2003, 107, 2294-2302) beschreiben, dass adulte Stamm- und Vorläuferzellen in der Lage sind, in Kardiomyozyten und Endothelzellen zu differenzieren. Sie könnten daher die Herzfunktion und Durchblutung bei ischämischen Herzerkrankungen verbessern. Es wird daher untersucht, ob Knochenmarkszellen sicher mit Hilfe eines Injektionskatheters in den Herzmuskel von Patienten injiziert werden können. Beschrieben wird dann die Injektion autologer mononukleärer Knochenmarkszellen direkt in den ischämischen Bereich der linken Herzkammer von Patienten mit koronarer Herzkrankheit im fortgeschrittenen Stadium. Die Vorläuferzellen werden aus dem Knochenmark über Dichtegradienten-zentrifugation isoliert und mit Hilfe von FACS Analyse anhand verschiedener Zelloberflächenmarker charakterisiert, aber nicht aufgetrennt. Dabei sind hämatopoetische Vorläuferzellen nach Tab. 3 des Dokuments dabei nur ein geringer Anteil der injizierten Zellen (ca. 2,5%), während T- und B-Zellen sowie Monozyten den Hauptanteil bilden. Als hämatopoetische Vorläuferzellen werden CD34⁺ CD45^{lo} Zellen bzw. CD133⁺ Cd45^{lo} Zellen beschrieben. Die mononukleären Knochenmarkszellen werden auf "viability" und "clonogenic capacity" hin untersucht. Die Knochenmarkszellen werden in Kochsalzlösung mit Heparin und 5% humanem Serum-Albumin injiziert. Die Möglichkeit einer Stratifizierung von Patienten auf Basis einer Analyse von Knochenmarks-Vorläuferzellen (BMP) und/oder Blut-abgeleiteten zirkulierenden Vorläuferzellen (BDP) wird jedoch ebenfalls nicht erwähnt. Es werden zudem keine isolierten endothelialen Vorläuferzellen, sondern nicht weiter fraktionierte mononukleäre Knochenmarkszellen insgesamt verwendet.

Kawamoto et al. Circulation 2003,107, 461-468 beschreiben, dass endotheliale Vorläuferzellen (EPCs) aus peripherem Blut als CD34⁺ mononukleäre Zellen isoliert werden können. Bei ischämischen Erkrankungen werden EPCs aus dem Knochenmark mobilisiert, siedeln sich im Bereich beginnender Neovaskularisierung an und differenzieren zu reifen Endothelzellen. Es wird daher untersucht, ob die Transplantation von Vorläuferzellen direkt in den Herzmuskel die Neovaskularisierung in ischämischem Gewebe fördert. Die Infusion autologer Vorläuferzellen direkt in den Herzmuskel erhöhte die Neovaskularisierung in Schweine-Ischämie-Modellen. Weiterhin wurden humane Vorläuferzellen heterolog in ischämische Herzregion von Ratten infundiert. Dazu werden humane mononukleäre CD34⁺ periphere Blutzellen als EPC-angereicheerte Zellfraktion verwendet.

Aicher et al. (Circulation 2003, 107, 2134-2139) beschreiben die Ansiedlung von radioaktiv markierten EPCs in verschiedenen Geweben nach Infusion in Ratten. Die Publikation beschreibt dafür ein System, in dem humane EPCs, die von gesunden Spendern stammen, in Ratten xeno-transplantiert werden, in denen zuvor ein Infarkt operativ induziert wurde, bzw. in Kontrolltieren ohne Infarkt. Die Isolierung der EPCs wird nicht beschrieben und die Klassifizierung als CD34⁺ periphere Blutzellen wird nicht genannt. Die Zellen werden kultiviert, d.h. expandiert und anschließend radioaktiv mit ¹¹¹Indium Oxin markiert, um die Verteilung der EPCs im Empfängerorganismus nach Infusion untersuchen zu können. Ebenfalls wird untersucht, ob sich die funktionelle Aktivität der Zellen nach radioaktiver Markierung von nichtmarkierten Zellen unterscheidet. Diese funktionelle Aktivität wird anhand eines Migrationstests untersucht, d.h. anhand der Wanderung der Zellen in Richtung des Wachstumsfaktors VEGF. Es wird gezeigt, dass sich nur ca. 1% der gesamten infundierten Zellen im Herz ansiedeln. Der Fachmann geht also davon aus, dass die migratorische Fähigkeit der EPCs mit der Ansiedlung der Zellen in Zielgeweben in einem Zusammenhang steht, möglicherweise auch im Infarktbereich. Es war aber nicht naheliegend, diese "Qualitätskontrolle" auch mit nicht radioaktiv markierten Zellen durchzuführen, zumal in der Publikation nicht-markierte Zellen als Referenz verwendet wurden.

Zuletzt beschreiben Wolfrum et al. (Arterioscler Thromb Vasc Biol 2003, 23, 729-736) allgemein die Wirkung von Statinen auf Endothelzellen.

Bevorzugt ist ein erfindungsgemäßes Verfahren, das weiterhin den Vergleich des aus der untersuchten Probe erhaltenen Ergebnisses mit einem Referenzwert und/oder dem Ergebnis einer Referenzprobe umfasst. Solche Referenzwerte können entweder von dem individuell zu behandelnden Patienten im Laufe der Therapie gewonnen werden oder sind das Ergebnis von großflächigen Studien. Üblicherweise stammt ein Referenzwert aus einer so genannten gesunden Kontrollgruppe. Der Fachmann in diesem Bereich ist ohne weiteres in der Lage, geeignete Studien Protokolle auszuwählen, um so geeignete Referenzwerte erhalten zu können.

Bevorzugt ist ein erfindungsgemäßes Verfahren, wobei die zu untersuchende Probe aus dem Menschen stammt. Dabei kann die zu untersuchende Probe erfindungsgemäß ausgewählt sein aus der Gruppe bestehend aus Knochenmark, peripherem Blut oder Fraktionen davon und Zellkultur-Suspensionen oder Fraktionen davon. Die Auswahl der einzelnen Probe hängt von der Art der zu untersuchenden Vorläuferzellen und dem Verfahren zur Ermittlung der Eigenschaften der Vorläuferzellen ab. Weiter bevorzugt wird im Falle der Entnahme von Blut dem peripheren Blut ein Gerinnungshemmer, insbesondere Heparin oder EDTA, zugesetzt. Dies erleichtert weitere Verarbeitungsschritte. In einem weiteren Aspekts der vorliegenden Erfindung wird die zu untersuchende Probe mittels Punktion aus dem Knochenmark entnommen. Diese Entnahme stellt die Grundlage für die Gewinnung von BMP dar. Die weitere Aufbereitung kann erfindungsgemäß eine Isolierung unter Verwendung von Dichte-Gradienten-Zentrifugation und/oder immunologischer Methoden, z.B. immunmagnetischer Verfahren, insbesondere unter der Verwendung von FACS umfassen. Dabei ist es erfindungsgemässe weiter bevorzugt, dass der für die BMP ausgewählte zellspezifische Oberflächenmarker ausgewählt ist aus CD34, CD45 und/oder CD133, und der für BDP ausgewählte zellspezifische Oberflächenmarker ausgewählt ist aus VEGFR2, CD105, vWF und/oder CD31.

Gemäß eines bevorzugten Verfahrens der vorliegenden Erfindung wird der Migrationstest in einer Boyden-Kammer oder einer modifizierten Version davon durchgeführt. Dem Fachmann wird jedoch ersichtlich sein, dass weitere Verfahren zur Ermittlung der migratorischen Kapazität verwendet werden können, ohne den Bereich der vorliegenden Erfindung zu verlassen. Dies betrifft insbesondere Ausführungsformen des Migrationstests, die entweder für die Automatisierung des Tests und/oder dessen Durchführung in einem "Point-of care"-Test angepasst sind.

Besonders bevorzugt ist ein erfindungsgemässes Verfahren, wobei der Migrationstest unter der Verwendung von SDF-1 oder VEGF als Chemo-Attraktant für BMP oder BDP durchgeführt wird. Wie in den beigefügten Versuchen gesehen wird, ist ein bevorzugter Stimulator für BMP SDF-1 und VEGF für BDP. Es können auch PIGF oder MCP-1 verwendet werden.

Voermans et al. (Voermans C, et al. Exp Haematol 2001 Dec;29(12):1456-64) beschreiben die Rolle von SDF-1-induzierter Migration von hämatopoetischen Vorläuferzellen (CD34(+)-Zellen) und einen entsprechenden migratorischen Test.

Eine Voraussetzung für den Erfolg einer Zelltherapie ist das so genannte "Homing", also die gezielte Wanderung von Zellen zur Ansiedlung in erwünschten Zielbereichen, insbesondere, falls eine intravaskuläre Verabreichungsroute gewählt wird. Daher wurde von den Erfindern angenommen, dass die migratorische Kapazität von adulten Vorläuferzellen in Richtung ihrer physiologischen Chemo-Attraktanten ihre Fähigkeit widerspiegeln könnte, in den Infarktbereich hinein zu wandern. In der Tat korreliert die Einwanderung von transplantierten Zellen in das betroffene Gewebe und die Verbesserung der Neovaskularisierung, die durch die intravenöse Infusion von menschlichen Vorläuferzellen induziert wurde, eng mit der z.B. durch SDF-1 induzierten migratorischen Kapazität für BMP und der durch VEGF induzierten migratorischen Kapazität für BDP. Dies waren jedoch bisher unbelegte Vermutungen, die erst durch die im Rahmen der vorliegenden Erfindung durchgeführten Versuche bestätigt worden. Es war weiterhin unerwartet, dass sich diese migratorische Kapazität als unabhängiger Vorhersage-Faktor so gut eignet, dass darauf basierende Diagnostika eine Verbesserung bei der Behandlung von CAD mit sich bringen würden. Zusammengenommen offenbart die Verbesserung der lokalen kontraktiven Myokardfunktion in Abhängigkeit der in Assoziation mit der funktionellen Aktivität der infundierten Vorläuferzellen eine kausale Beziehung zwischen der migratorischen Kapazität von Vorläuferzellen und dem Erfolg einer Zelltherapie in Patienten mit akutem Myokardinfarkt. Die vorliegende Beschreibung offenbart daher ein neues diagnostisches Verfahren, mit dessen Hilfe eine Verbesserung/Optimierung der Zelltherapie bei Patienten mit kardiovaskulären Erkrankungen ermöglicht wird. Das Verfahren erlaubt anhand der Bestimmung der funktionellen Aktivität der Vorläuferzellen, zuverlässige Vorhersagen zu machen, ob der jeweilige Patient von einer Zelltherapie (Transplantation der eigenen von Vorläuferzellen) zur Regeneration des zerstörten geschädigten Herzmuskelgewebes profitieren wird oder ob diese Therapie wahrscheinlich keinen Erfolg haben wird, weil die Zellen eine zu geringe Fähigkeit haben, sich im Zielgewebe anzusiedeln.

Die offenbarten Verfahren können immer ein der Diagnose und/oder Therapie aller kardiovaskulären Erkrankungen angewendet werden, bei denen von einer Transplantation von Vorläuferzellen ein therapeutischer Vorteil erwartet wird. Bevorzugt ist ein erfindungsgemässes Verfahren, wobei die kardiovaskulären Erkrankungen ausgewählt sind aus der Gruppe bestehend aus stabiler und instabiler Angina, stabiler koronarer Herzerkrankung, akutem Koronarsyndrom, Myokardinfarkt, akutem Myokardinfarkt, akutem Herzsyndrom, koronarer Arterienerkrankung, chronischer ischämischer Kardiomyopathie (ICMP), dilatativer Kardiomyopathie (DCM), Herzinsuffizienz oder anderen Ursachen einer Herzschwäche.

Gemäß eines weiteren Aspekts der vorliegenden Offenbarung kann das beschriebene Verfahren unmittelbar vor einer Zellinfusion in den Säuger durchgeführt werden. Wie bereits oben ausgeführt können durch den beschriebenen Test solche Spender effektiv ausgeschlossen werden und gleichzeitig Patienten mit einem höheren Risiko identifiziert werden, die schlechtere Neovaskularisierungskapazitäten aufweisen. Diese Patienten können dann geeignet und insbesondere präventiv und/oder unterstützend behandelt werden. Alternativ können durch diesen Test auch Patienten identifiziert werden, die durch eine Vorläuferzell-Therapie einen erhöhten Nutzen erfahren können. Dies betrifft in einem weiteren Aspekt des Verfahrens gemäß der vorliegenden Erfindung eine Therapie, bei der die untersuchten isolierten BMP und/oder BDP für den zu behandelnden Patienten autolog sind.

Ein weiterer Aspekt der vorliegenden Beschreibung betrifft einen diagnostischen Kit, der neben Mitteln zur Durchführung der genannten erfindungsgemäßen Verfahren gegebenenfalls weitere geeignete Komponenten und/oder Hilfsstoffe enthält. Das Kit kann aus einem oder mehreren getrennten Behältern bestehen. Geeignete Komponenten können insbesondere Gebrauchsanweisungen oder Farbtafeln zum Abgleich von Messergebnissen anhand von Referenzwerten sein.

Beschreibungsgemäß wird der Kit vorzugsweise zur Diagnose und/oder der Prognose von kardiovaskulären Erkrankungen, zur Überwachung von deren Therapie und/oder zur Stratifizierung für eine geplante Zelltherapie mit Stamm- und Vorläuferzellen zur Steigerung der Perfusion von ischämischem Gewebe bzw. zur Regeneration von Gewebeverlust, wie zum Beispiel bei Herzinsuffizienz, verwendet.

Ein weiterer Aspekt der vorliegenden Erfindung betrifft dann ein *in vitro* Verfahren zur Isolierung von spezifischen Knochenmarks-Vorläuferzellen (BMP) und/oder Blut-abgeleiteten zirkulierender Vorläuferzellen (BDP), wobei das Verfahren die Schritte umfasst von a) Entnehmen einer Probe aus einem Säugetier-Spender, b) Isolieren von (BMP) und/oder (BDP) mittels zellspezifischer Oberflächenmarker aus der so erhaltenen Probe, und c) Überprüfung der kardiovaskulären Funktionalität der isolierten BMP und/oder BDP mittels eines geeigneten Migrationstests. Erfindungsgemäß bevorzugt ist es, dass die zu untersuchende Probe aus einem Säuger, insbesondere dem Menschen stammt.

Wie oben bereits erwähnt, kann das Verfahren an allen aus einem Spender gewonnenen Stamm- und Vorläuferzellpopulationen probenunabhängig von der Isolierungsmethode durchgeführt werden, von denen vermutet wird, dass diese geeignete BMP oder BDP enthalten. Bevorzugt ist ein Verfahren, wobei die zu untersuchende Probe ausgewählt ist aus der Gruppe bestehend aus Knochenmark, peripherem Blut oder Fraktionen davon und Zellkultur-Suspensionen oder Fraktionen davon. Insbesondere bevorzugt ist ein Verfahren, wobei die zu untersuchende Probe auf geeignete Weise aus dem Knochenmark entnommen wird. Bevorzugt ist, dass die zu untersuchende Probe mittels Punktion aus dem Knochenmark entnommen wird.

Auf Grund der Tatsache, dass bei einer Entnahme meist ein heterogenes Gemisch von verschiedenen Vorläuferzellen erhalten wird, umfasst ein bevorzugtes Verfahren die Isolierung unter Verwendung von Dichte-Gradienten-Zentrifugation und/oder immunologischer Methoden. Eine besonders bevorzugtes Isolierungsverfahren erfolgt unter der Verwendung von FACS oder immunomagnetischer Separation, wobei der zellspezifische Oberflächenmarker für BMP ausgewählt sein kann aus CD34, CD45 und/oder CD133, und für BDP ausgewählt sein kann aus VEGFR2, CD105, vWF und/oder CD31.

Wie bereits oben erwähnt, wird in einem weiteren Aspekts der vorliegenden Erfindung der Migrationstest in einer Boyden-Kammer oder in einer Weiterentwicklung davon (siehe oben) durchgeführt. Auch hier ist es bevorzugt, dass der für den Migrationstest verwendete Chemo-Attraktant SDF-1, VEGF, P1GF oder MCP-1 ist.

Ein wesentlicher Aspekt der vorliegenden Erfindung ist die Ermittlung der kardiovaskulären Funktionalität von Vorläuferzellen, die aus einem Säugetier-Spender entnommen wurden. Auf Basis der mittels des erfindungsgemäßen Verfahrens gewonnenen Erkenntnisse über die Brauchbarkeit und Effizienz der gewonnenen Vorläuferzellen für die Zelltherapie kann diese Therapie auf den individuellen Patienten angepasst werden. Wie bereits oben angegeben, wird vermutet, dass im Knochenmark bestimmter Spender oder Patienten, insbesondere bei Patienten mit ICMB, eine Störung vorhanden ist, die die kardiovaskuläre Funktionalität der Vorläuferzellen beeinträchtigt. Um in solchen Fällen gegen diese Beeinträchtigung "gegensteuern" zu können, beschreibt die vorliegenden Offenbarung in einem weiteren Aspekts ein Verfahren, wobei die isolierten BMP und/oder BDP weiter genetisch modifiziert werden, um die kardiovaskuläre Funktionalität der Zellen zu verbessern. Verfahren, um die BMP und/oder BDP genetisch zu modifizieren, und dazu geeignete genetische Konstrukte sind dem Fachmann gut bekannt und können leicht durch ihn hergestellt und verwendet werden. Die genauen genetischen Mechanismen der Beeinträchtigung der kardiovaskulären Funktionalität bei bestimmten Patienten sind bisher nicht bekannt, jedoch ergaben die Experimente der vorliegenden Offenbarung, dass die Migration der aus diesen Spendern stammenden Vorläuferzellen beeinträchtigt ist. Um dieser Beeinträchtigung entgegenwirken zu können, wäre es ein Ansatz, die Vorläuferzellen mit denjenigen Rezeptoren zu transfizieren, die für die jeweiligen Chemo-Attraktanten (insbesondere SDF-1 für BMP und VEGF für BDP) spezifisch sind. So wäre ein Ansatz die Transfektion von BMP mit Expressionskonstrukten von CXCR4 oder in die Regulation von CXCR4 eingreifende Konstrukte, von dem vermutet wurde, dass er in beeinträchtigten BMP herunter reguliert vorliegen könnte. Ein anderer Ansatz wäre die Modifikation von Integrinen, wie zum Beispiel CD49a oder anderen Faktoren, wie zum Beispiel VCAM-1 oder damit interagierender Faktoren.

Ein weiterer Aspekt beschreibt dann eine spezifische Knochenmarks-Vorläuferzelle (BMP) oder Blut-abgeleitete zirkulierende Vorläuferzelle (BDP), die mittels eines obengenannten Verfahrens hergestellt worden ist. Diese spezifische Knochenmarks-Vorläuferzelle (BMP) oder Blut-abgeleitete zirkulierende Vorläuferzelle (BDP) gemäß der vorliegenden Offenbarung kann als isolierte BMP und/oder isolierte BDP für den Säuger/Patienten (also den jeweiligen Empfänger) autolog (eigen) sein. Möglich sind natürlich auch Gemische der Zellen.

Wie oben bereits erwähnt, können die isolierten BMP und/oder BDP im Rahmen der Zelltherapie verwendet werden. Weiterhin beschrieben wird somit ein Verfahren zur Herstellung einer pharmazeutischen Zusammensetzung, das zunächst ein oben genanntes erfindungsgemäßes Verfahren und weiterhin ein Formulieren der pharmazeutischen Zusammensetzung durch Mischen mit herkömmlichen pharmazeutisch akzeptablen Trägern und/oder Verdünnungsmitteln umfasst. Entsprechend geeignete Träger und/oder Verdünnungsmittel sind dem Fachmann bestens bekannt und können sehr leicht an die jeweilige spezifische Applikationen angepasst werden. Bevorzugt werden die Vorläuferzellen jenseits des vorher implantierten Stents infundiert.

Die Formulierung kann weiterhin im Gemisch mit weiteren pharmazeutisch aktiven Substanzen vorliegen. Bevorzugt sind dabei Substanzen, die ausgewählt sind aus Statinen, Erythropoetin und/oder VEGF. Denkbar sind auch andere Substanzen, solange diese die kardiovaskuläre Funktionalität der verabreichten Vorläuferzellen nicht nachteilig beeinträchtigen.

Zuletzt betrifft die vorliegende Beschreibung die Verwendung einer pharmazeutischen Zusammensetzung zur Behandlung von kardiovaskulären Erkrankungen, ausgewählt aus der Gruppe bestehend aus stabiler und instabiler Angina, stabiler koronarer Herzerkrankung, akutem Koronarsyndrom, Myokardinfarkt, akutem Myokardinfarkt, akutem Herzsyndrom, koronarer Arterienerkrankung, chronischer ischämischer Kardiomyopathie (ICMP), dilatativer Kardiomyopathie (DCM), Herzinsuffizienz oder anderen Ursachen einer Herzschwäche. Beschrieben ist dabei eine Verwendung, bei der die Behandlung die Zellinfusion in den Säuger umfasst. Dabei können auch, wie oben genannt, andere pharmazeutisch aktive Substanzen verabreicht werden, insbesondere bevorzugt umfasst die Behandlung weiterhin die Verabreichung von Statinen, insbesondere VEGF, und/oder Erythropoetin.

Vor kurzem durchgeführte Experimente der Erfinder ergaben, dass die migratorische Kapazität der Vorläuferzellen von Patienten mit CAD sehr eng mit der neovaskulären Verbesserung nach der Transplantation in Nacktmäuse nach Hinterlaufischämie korrelierte (r = 0,783; p = 0,001; n = 11). Daher konzentrierten sich die Erfinder auf die Messung migratorischen Kapazität der transplantierten Vorläuferzellen unmittelbar vor der intrakoronaren Infusion in Patienten mit akutem myokardialem Infarkt, um die funktionellen Charakteristika der transplantierten Vorläuferzellen mit quantitativen Ergebnissen des Ausgangs nach 4 Monaten in Zusammenhang zu bringen. Die Ergebnisse zeigten, dass eine hohe migratorische Kapazität der infundierten Vorläuferzellen eine Verbesserung der links-ventikulären Ventrikel-Pumpfunktion und eine Infarktgrössenverringerung vorhersagte, was einen kausalen Zusammenhang zwischen der Migrationsfähigkeit der Vorläuferzellen und der Regeneration der Herzfunktion nach einer Zelltherapie in Patienten mit akutem Myokardialinfarkt mit geschädigtem Myokard vermuten lässt.

Die vorliegende Erfindung zeigt somit zum ersten Mal, dass die funktionelle Kapazität der transplantierten Vorläuferzellen eine hauptsächliche unabhängige Determinante der anschliessenden Verbesserung bei der regionalen linken Ventrikelfunktion ist und eine InfarktGrössenverringerung nach der intrakoronaren Zelltransplantation vorhersagt. Interessanter weise war die funktionelle Aktivität der Zellen, wie durch ihre migratorische Aktivität ermittelt, informativer, als die Zellzahl. Zusammengefasst beschreiben diese Daten zum ersten Mal einen kausalen Zusammenhang zwischen Vorläufer-Zelltherapie und der Verbesserung der links-ventikulären Pumpfunktion/Ventrikelregeneration in Patienten nach Myokardschädigung.

Vorhergehende experimentelle Untersuchungen lassen vermuten, dass die Verbesserung in der Ventrikelfunktion nach experimentell induziertem Myokardinfarkt an einer stimulierten Neoangiogenese liegt, die ein spätes myokardiales Remodelling durch verstärkten myokardialen Blutfluss, Rettung von ruhendem Myokard, die Verringerung der myokardialen Fibrose und eine verringerte Apoptoserate von hypertrophierten Myocyten in der peri-Infarktregion bewirkt (Kawamoto A, Gwon HC, Iwaguro H, Yamaguchi Jl, Uchida S, Masuda H, Silver M, Ma H, Kearney M, Isner JM, Asahara T. Therapeutic potential of ex vivo expanded endothelial progenitor cells for myocardial ischemia. Circulation. 2001; 103:634-7., Kocher AA, Schuster MD, Szabolcs MJ, Takuma S, Burkhoff D, Wang J, Homma S, Edwards NM, Itescu S. Neovascularization of ischemic myocardium by human bone-marrow-derived angioblasts prevents cardiomyocyte apoptosis, reduces remodeling and improves cardiac function. Nat Med. 2001:7:430-6., Takahashi T, Kalka C, Masuda H, Chen D, Silver M, Kearney M, Magner M, Isner JM, Asahara T. Ischemia- and cytokine-induced mobilization of bone marrow-derived endothelial progenitor cells for neovascularization. Nat Med. 1999:5:434-8., Murohara T, Ikeda H, Duan J, Shintani S, Sasaki K, Eguchi H, Onitsuka I, Matsui K, Imaizumi T. Transplanted cord blood-derived endothelial precursor cells augment postnatal neovascularization. J Clin Invest. 2000:105:1527-36.). Zusätzlich wurde berichtet, dass die intramyokardiale Injektion von BMP zur Regeneration von signifikanten Mengen von sich kontraktierendem Myokard führte, was vermuten lässt, dass die neue Erzeugung von Myokard zu einer Verbesserung des Ausgangs von Myokardinfarkt im Anschluss an die lokale Zufuhr von adulten Vorläuferzellen führen kann. In der Tat konnte von den Erfindern kürzlich gezeigt werden, dass BDP ihre Fähigkeit beibehalten, sich in funktionelle kardiale Myocyten zu transdifferenzieren (Badorff C, Brandes RP, Popp R, Rupp S, Urbich C, Aicher A, Fleming I, Busse R, Zeiher AM, Dimmeler S. Transdifferentiation of blood-derived human adult endothelial progenitor cells into functionally active cardiomyocytes. Circulation. 2003:107:1024-32.). Die gilt auch für BMP (Orlic D, Kajstura J, Chimenti S, Jakoniuk I, Anderson SM, Li B, Picket J, McKay R, Nadal-Ginard B, Bodine DM, Leri A, Anversa P. Bone marrow cells regenerate infarcted myocardium. Nature. 2001 ;410:701 -5.).

Offensichtlich kann die vorliegende Studie nicht die zellulären Mechanismen offenbaren, die mit den verbesserten linken Ventrikel Remodelling-Prozessen assoziiert sind, die im Anschluss an einer Vorläuferzellen-Therapie stattfinden. Jedoch zeigte eine kürzliche Studie, die in Patienten mit Hinterlaufischämie durchgeführt wurde, dass die intramuskuläre Injektion von Vorläuferzellen in den gastrocnemischen Muskel zu einer signifikanten Verbesserung bei der Hinterlauf-Perfusion führte (Tateishi-Yuyama E, Matsubara H, Murohara T, Ikeda U, Shintani S, Masaki H, Amano K, Kishimoto Y, Yoshimoto K, Akashi H, Shimada K, Iwasaka T, Imaizumi T. Therapeutic angiogenesis for patients with limb ischaemia by autologous transplantation of bone-marrow cells; a pilot study and a randomised controlled trial. Lancet. 2002;360:427-35.). In Übereinstimmung damit, dass eine intrakoronare Infusion von Vorläuferzellen mit einem verbesserten Perfusionsindex im Zielbereich assoziiert war, zeigen diese Daten an, dass die Ansiedlung der Vorläuferzellen im Infarktbereich zu einer verbesserten Neovaskularisierung führen kann, die zu einer Verringerung der LV-Dilatation und einem Erhalt der kontraktilen Performance durch die Rettung von ruhendem Myokard, der Verringerung von myokärdialer Fibrose und Apoptose in der peri-Infarktregion führen kann. Zusätzlich können eingewanderte Vorläuferzellen nicht nur eine Vielzahl von angiogenetischen Wachstumsfaktoren freisetzen, wodurch endogene Neovaskularisierungsprozesse in der Infarkt Grenzzone verstärkt werden, sondern auch Faktoren freisetzen, die zirkulierende oder Gewebe-residente Herz-Vorläuferzellen anlocken, wodurch endogene Reparaturmechanismen des Myokards verstärkt werden. Zuletzt, obwohl die Zahl von infundierten Vorläuferzellen nicht die anschliessende funktionelle Verbesserung vorhersagte, können wir die Möglichkeit nicht ausschliessen, dass angesiedelte Vorläuferzellen sich möglicherweise zu funktionell aktiven Kardiomyocyten differenziert haben, was zu einer Regeneration von signifikanten Mengen von sich kontrahierendem Myokard führen würde. Vergleiche zwischen der Transplantation von kontraktilen gegenüber nicht-kontraktilen Zellen in experimentellen Studien zeigten, dass die Verbesserung der ventrikulären Kontraktion spezifischer von kontraktilen Zellen abhängig war, wohingegen die ventrikuläre Dilatation unabhängig von den Zelltypen verbessert war (Hutcheson KA, Atkins BZ, Hueman MT, Hopkins MB, Glower DD, Taylor DA. Comparison of benefits on myocardial performance of cellular cardiomyoplasty with skeletal myoblasfs and fibroblasts. Cell Transplant. 2000;9:359-68., Sakai T, Li RK, Weisel RD, Mickle DA, Jia ZQ, Tomita S, Kirn EJ, Yau TM. Fetal cell transplantation: a comparison of three cell types. J Thorac Cardiovasc Surg. 1999; 118:715-24.). In der vorliegenden Studie war die systolische Funktion deutlich verbessert, was mit den experimentellen Ergebnissen nach der Transplantation von Knochenmark-abgeleiteten Angioblasten übereinstimmt (Kocher AA, Schuster MD, Szabolcs MJ, Takuma S, Burkhoff D, Wang J, Homma S, Edwards NM, Itescu S. Neovascularization of ischemic myocardium by human bone-marrow-derived angioblasts prevents cardiomyocyte apoptosis, reduces remodeling and improves cardiac function. Nat Med. 2001:7:430-6.).

Zusammengefasst offenbart die Beschreibung die migratorische Kapazität der Vorläuferzellen als eine hauptsächliche und unabhängige Determinante, um eine Verbesserung der Herzfunktion und Verringerung der Infarktgröße im Anschluß an die intrakoronare Infusion von adulten Vorläuferzellen nach Zelltherapie vorherzusagen. Darüber hinaus ermöglicht die Erfindung auch eine Verbesserung der funktionellen Aktivität von isolierten Vorläuferzellen vor einer Transplantation, um so die kardiale Regeneration nach ischämischer Verletzung durch eine Zelltherapie weiter zu verbessern.

Die Daten der vorliegenden Studie zeigen weiter, dass mononukleare Zellen, die aus dem Knochenmark von Patienten mit ischämischer Kardiomyopathie isoliert wurden, eine signifikant verringerte Aktivität bei der Unterstützung der Neovaskularisierung in Nacktmäusen nach der Induktion von Interrupt-Hinterlaufischämie aufweisen. So war die funktioneller Kapazität von BMP, bestimmt durch ihre koloniebildende Aktivität und ihre migratorische Antwort auf SDF-1 in BMP, die aus Patienten mit ICMP isoliert wurden signifikant verringert. Die Injektion dieser BMP aus Patienten mit ICMP in Mäuse mit Hinterlaufischämiezeigte eine signifikant verringerte Fähigkeit, die Hinterlauf-Perfusion nach unilateraler Ligation der den femoralen Arterie wiederherzustellen. Es kann daraus geschlossen werden, dass die verringerte funktionelle Aktivität das Neovaskularisierungs-Potential von BMP aus Patienten mit ICMP begrenzt. Die funktionelle Beeinträchtigung von BMP aus Patienten mit ICMP kann auch deren therapeutisches Potential für die medizinische Zelltherapie begrenzen, insbesondere wenn eine intravaskuläre Route der Verabreichung verwendet wird, die erfordert, dass die Vorläuferzellen gegen einen Gradienten eines Chemo-Attraktanten extravasieren, um in i-schämisches Gewebe einzuwandern. Die Überwachung der migratorischen und koloniebildenden Aktivität von Vorläuferzellen vor der Therapie kann als ein Surrogat zu Identifizierung von Patienten dienen, die einen grösseren Vorteil aus der Zelltherapie herausziehen könnten. Auf der anderen Seite lässt eine kürzliche experimentelle Studie vermuten, dass voll funktionsfähige BMPs aus gesunden Spendermäusen die altersbedingte verminderte Angiogenese in einem Mausmodell wieder herstellen können. Daher erscheint es vielversprechend, dass eine pharmakologische oder genetische Manipulation der funktionell beeinträchtigten BMPs vor einer Infusion ihre funktionelle Aktivität verbessern kann und anschliessend den therapeutischen Vorteil des Patienten aus der Therapie verbessern kann.

Die Erfindung soll nun in den folgenden Beispielen unter Bezug auf die beigefügten Figuren näher beschrieben werden, ohne jedoch auf diese beschränkt zu werden. Es zeigt in den Figuren
Figur 1 die quantitative linke ventrikuläre Angiographie, die die Ausstossfraktion (A), End-systolische und End-diastolische Volumina (B), bei Basislinie und Nachfolge für Patienten, die zirkulierende Blut-abgeleitete (BDP) oder Knochenmark-abgeleitete (BMP) Vorläuferzellen enthielten und für Patienten der nicht-randomisierten Kontrollgruppe zeigt,

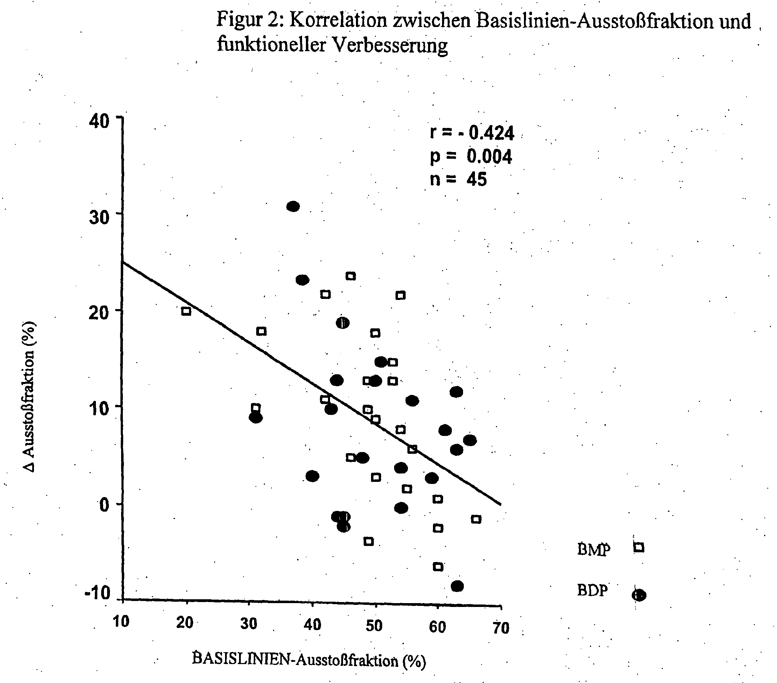

Figur 2 die Korrelation zwischen Basislinien linker ventrikulärer Ausstossfraktion und die Verbesserung in der Ausstossfraktion mit der Regressionslinie für die gesamte Studiengruppe zeigt,
Figur 3 den Mittelwert der funktionellen Verbesserung und der Infraktgrössenverringerung für Patienten zeigt, die Vorläuferzellen entweder mit niedriger oder hoher migratorischer Kapazität zeigt.
Figur 4 die Flusszytometrische Analyse auf Vorläuferzellmarker in BMP aus den gesunden Kontrollen und Patienten mit stabiler ischämischer Kardiomyopathie. a-c, keine signifikanten Unterschiede wurden zwischen den zwei Gruppen beobachtet, und
Figur 5 die funktionelle Aktivität der BMP aus den gesunden Kontrollen und Patienten mit stabiler ischämischer Kardiomyopathie, a, die koloniebildenden Einheiten pro 5 x 10⁵ BMPs wurden nach 14 Tagen bestimmt, b, die migratorische Kapazität der BMP in Antwort auf stromale Zellen-abgeleiteten Faktor 1 (SDF-1) und c, vaskulärer Endothel Wachstumsfaktor (VEGF) wurde unter der Verwendung des BioCoat® Invasionstests gemessen, d, Korrelation zwischen SDF-1 induzierter Migration und koloniebildender Kapazität und e, Korrelation zwischen VEGF induzierter Migration und koloniebildender Kapazität.

### Beispiele

### Patienten

Patienten zwischen 18 und 75 Jahren wurden in die Studie aufgenommen, falls sie einen ersten akuten ST-Erhöhungs myokardialen Infarkt hatten, der akut durch Koronarstent mit GPIIb/IIIa Blockade behandelt wurde. Ausschlusskriterien waren die Anwesenheit von kardiogenem Schock (definiert als systolischer Blutdruck < 80 mmHg, was intravenöse Pressoren oder intra-Aorta Ballon-Gegenpuls erforderte), starke Blutungen, die Bluttransfusion nach akuter Reperfusionsbehandlung erforderten, Historie von Leukopenie, Thrombocytopenie, heaptischer oder renaler Dysfunktion, Anzeichen maligner Erkrankungen oder Unwillen zur Teilnahme. Das Ethikkomitee des Universitätsklinikum der Johann Wolfgang Goethe Universität in Frankfurt, DE, genehmigte das Protokoll der durchgeführten Studie und die Studie wurde in Übereinstimmung mit der Erklärung von Helsinki durchgeführt. Eine schriftliche, informierte Zustimmung wurde von jedem Patienten erhalten.

Als eine interne Referenzgruppe, die den Standard der Pflege widerspiegelte, die am Krankenhaus verabreicht wurde, wurden 15 Patienten ausgewählt, die auf Ausstossfraktion, Infarktort und Infarktgrösse mit der Stduienpopulation abgeglichen wurde, in denen eine akute Reperfusionstherapie durch Stent-Implantation durchgeführt wurde und für die akute und 4 Monate Nachfolge-gepaarte LV-Angiogramme erhältlich waren.

### Protokoll der durchgeführten Studie

Das Studienprotokoll wurde früher beschreiben (Assmus B, Schachinger V, Teupe C, Britten M, Lehmann R, Dobert N, Grunwald F, AicherA, Urbich C, Martin H, Hoelzer D, Dimmeler S, ZeiherAM. Transplantation of Progenitor Cells and Regeneration Enhancement in Acute Myocardial Infarction (TOPCARE-AMI). Circulation. 2002;106:3009-17.). Kurz gesagt wurden Patienten zufällig zum Empfang einer interkoronaren Infusion von entweder Knochenmarks-Vorläuferzellen (BMP) und/oder Blut-abgeleiteter zirkulierender Vorläuferzellen (BDP) 4 Tage nach AMI zugeordnet.

Bei Patienten, die Knochenmarks-Vorläuferzellen (BMP) erhielten, wurden am Morgen des Tages der Zelltransplantation 50ml Knochenmarks-Aspirat erhalten. Knochenmarksabgeleitete mononukleare Zellen (BMCs) wurden durch Dichtegradienten-Zentrifugation erhalten. Nach zwei Waschschritten wurden die Zellen in 10 ml X-vivo 10 Medium (Biowhittaker) resuspendiert. Die Zellsuspension bestand aus heterogenen Zellpopulationen, die neben hämatopoetischen Vorläuferzellen, die durch FACS-Analyse unter der Verwendung von direkt konjugierten Antikörpern gegen anti-Mensch CD34 (FITC, Becton Dickinson), anti-CD45 (Becton Dickinson), CD 14 (BD Pharmingen, San Dieago, US), CXCR4 (BD Pharmingen), CD49d (BD Pharmingen) und CD133 (Miltenyl Biotech, Bergisch Gladbach, DE) bestimmt wurden, auch andere Zelltypen enthielten (z.B. Nebenpopulations-Zellen, Stroma-Zellen, usw.). Insgesamt wurde ein Mittelwert von 5,5 ± 3,6 x 10⁶ CD34/CD45-positiven Zellen (ca. 2,5% in 219 ± 82 x 10⁶ mononuklearen Zellen) pro Patient infundiert.

Bei Patienten, die Blut-abgeleitete zirkulierende Vorläuferzellen (BDP) erhielten, wurden 250 ml unmittelbar nach der zufälligen Zuordnung (24 Stunden nach dem AMI) venöses Blut gesammelt. Mononuklearen Zellen wurden in X-vivo-15 Medium (Biowhittaker, Apen, DE), ergänzt mit 1 ng/ml Träger-freiem menschlichem rekombinantem VEGF (R&D, Wiesbaden, DE), 0,1 µM Atorvastatin (Pfizer, Freiburg, DE) und 20% menschlichem Serum, abgenommen von jedem einzelnen Patienten suspendiert. Die Zellen wurden bei einer Dichte von 6,4 x10⁵ Zellen/mm² auf Fibronektin-beschichteten Schalen (Roche, Grenzach. DE) ausgesät. Nach 3 Tagen Kultur wurden die Zelle mit 0,5 mM EDTA abgewaschen, zweimal gewaschen und in einem finalen Volumen von 10 ml X-vivo 10 Medium resuspendiert. Die erhaltenen Zellsuspension (Mittel der injizierten Zellen 17 ± 12 x 10⁶) enthielt eine heterogene Population von Vorläuferzellen. Jedoch zeigten mehr als 90% der Zellen endothele Charakteristika, wie durch Dil-acetylierte LDL-Aufnahme und Lektinbindung und die Expression von typischen Endothel-Markerproteinen einschliesslich VEGFR2 (KDR) (ReliaTech, Braunschweig, DE), Endoglin (CD105) (NeoMarkers, Asbach. DE), von Willebrandfaktor (vWF) (Oncogene, Schwalbach, DE) und Plättchen-Endothelzell-Adhäsionsmolekül-1 (PECAM-1/CD31) (Dianova, Hamburg) gezeigt wurde (Assmus B, Schachinger V, Teupe C, Britten M, Lehmann R, Dobert N, Grunwald F, AicherA, Urbich C, Martin H, Hoelzer D, Dimmeler S, ZeiherAM. Transplantation of Progenitor Cells and Regeneration Enhancement in Acute Myocardial Infarction (TOPCARE-AMI). Circulation. 2002;106:3009-17., Dimmeler S, Aicher A, Vasa M, Mildner-Rihm C, Adier K, Tiemann M, Rutten H, Fichtlscherer S, Martin H, Zeiher AM. HMG-CoA reductase inhibitors (statins) increase endothelial progenitor cells via the PI 3-kinase/Akt pathway. J din Invest. 2001;108:391-7., Vasa M, Fichtlscherer S, AicherA, AdIerK, Urbich C, Martin H, ZeiherAM, Dimmeler S. Number and migratory activity of circulating endothelial progenitor cells inversely correlate with risk factors for coronary artery disease. Circ Res. 2001;89:E1-7., Vasa M, Fichtlscherer S, Adier K, AicherA, Martin H, ZeiherAM, Dimmeler S. Increase in circulating endothelial progenitor cells by statin therapy in patients with stable coronary artery disease. Circulation. 2001 ,-103:2885-90.).

Die Zellen wurden über einen über-den-Draht Ballonkatheter infundiert, der in den vorher während des akuten Reperfusionsverfahrens implantierten Stent vorgeschoben wurde und mit niedrigem Druck aufgepumpt, um den Blutfluss für 3 Minuten vollständig zu blockieren um eine Adhäsion und potentielle Transmigration der infundierten Zellen durch das Endothel zu ermöglichen. Diese Manöver wurde 3 Mal wiederholt, um die Infusion der gesamten 10 ml Vorläuferzellen zu ermöglichen, unterbrochen von 3 Minuten Reflux durch Deflation des Ballons, um eine extensive Ischämie zu minimieren. Nach Vervollständigung der intrakoronaren Zelltransplantation wurde eine koronare Angiographie wiederholt, um die Gefässöffnung und einen ungehinderten Fluss von Kontrastmaterial sicherzustellen.

### Ermittlung der migratorischen Kapazität von transplantierten Vorläuferzellen

Unmittelbar vor intrakoronarer Zellinfusion wurde eine Probe von Vorläuferzellen in 500 µl Endothel-Basalmedium (EBM, Cell Systems) resuspendiert, gezählt und 2 ^{x} 104 Zellen wurden in die obere Kammer einer modifizierten Boyden-Kammer plaziert. Dann wurde die Kammer in eine 24-Tüpfel Kulturschale plaziert, die EBM und entweder 50 ng/ml VEGF zur Messung der migratorischen Kapazität von zirkulierenden Blut-abgeleiteten oder 100 ng/ml SDF-1 zur Messung der migratorischen Kapazität von Knochenmark-abgeleiteten Vorläuferzellen enthielt. Nach 24 Stunden Inkubation bei 37°C wurde die untere Seite des Filters mit PBS gewaschen und mit 2% Paraformaldehyd fixiert. Zur Messung wurden die Zellkerne mit DAPI gefärbt. Zellen, die in die untere Kammer wanderten wurden manuell in 5 zufälligen Mikroskopfeldern durch einen blinden Untersuchenden gezählt (Vasa M, Fichtlscherer S, AicherA, AdIerK, Urbich C, Martin H, ZeiherAM, Dimmeler S. Number and migratory activity of circulating endothelial progenitor cells inversely correlate with risk factors for coronary artery disease. Circ Res. 2001;89:E1-7., Asahara T, Takahashi T, Masuda H, Kalka C, Chen D, Iwaguro H, Inai Y, Silver M, Isner JM. VEGF contributes to postnatal neovascularization by mobilizing bone marrow-derived endothelial progenitor cells. Embo J. 1999; 18:3964-72.).

### Test auf koloniebildende Einheiten

BMPs (1 x 10⁵ pro Schale) wurden auf Methylcellulose-Platten (Methocult GF H4535, die Stammzellenfaktor, G-CSF, GM-CSF, Interleukin-3, Interleukin-6 enthielten, CellSystems, St. Katharinen, DE) ausgesät. Die Platten wurden unter Phasekontrastmikroskopie untersucht und koloniebildende Einheiten Granulocyten-Makrophage (CFU-GM; Kolonien) wurde nach 14 Tagen Inkubation durch zwei verschiedene Forscher gezählt.

### Linke-ventrikuläre Angiographie

LV-Angiogramme wurden gemäss Standard-Aufnahme-Richtlinien erhalten. Die LV-Ausstossfraktion und -Volumina wurden unter der Verwendung des Flächen-Längen-Verfahrens (Dodge HT Sh, Ballew DW, et al. The use of biplane angiography for the measurement of left ventricule volume in man. Eur Heart J. 1960;60:762-776.) berechnet, und die regionale Wandbewegung wurde mittels der Verwendung des Mittellinien-Chord-Verfahrens (Sheehan F, Bolson E, Dodge H, Mathey D, Schofer J, Woo H. Advantages and applications of the centerline method for characterizing regional ventricular function. Circulation. 1986;74:293-305.) bestimmt.

### Statistische Analyse

Kontinuierliche Variablen sind als Mittel ± SD dargestellt. Kategorische Variablen wurden mit dem Chi-Quadrat-Test oder Fischer's exaktem Test verglichen. Die statistischen Vergleiche zwischen anfänglichen und Nachfolgedaten wurden auf eine nicht-parametrische Weise unter der Verwendung des gepaarten Zeichen-Tests durchgeführt. Die lineare nicht-parametrische Korrelation wurde unter der Verwendung der Spearman-Korrelation berechnet. Die multivariante Analyse wurde unter der Verwendung des linearen Regressionsmodells durchgeführt. Es wurde eine statistische Signifikanz angenommen, falls p < 0,05. Alle statistischen Analysen wurde unter der Verwendung von SPSS (Version 11.0, SPSS Inc.) durchgeführt.

Die demographischen, klinischen und angiograpischen Charakteristika der Studienpopulation sind in Tabelle I gezeigt. Es gab keine signifikanten Unterschiede in irgendeinem der Basislinien-Parameter.

### Effekte der Vorläufer-Zelltherapie auf die LV Funktion

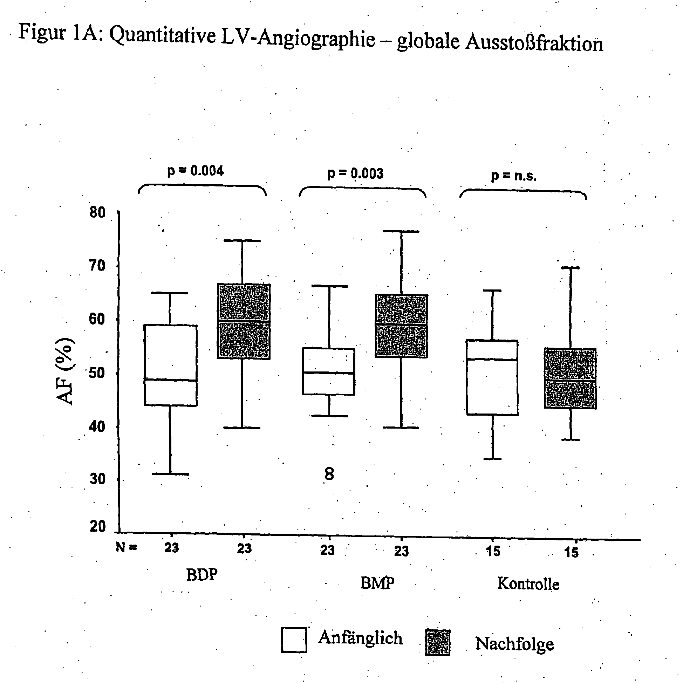

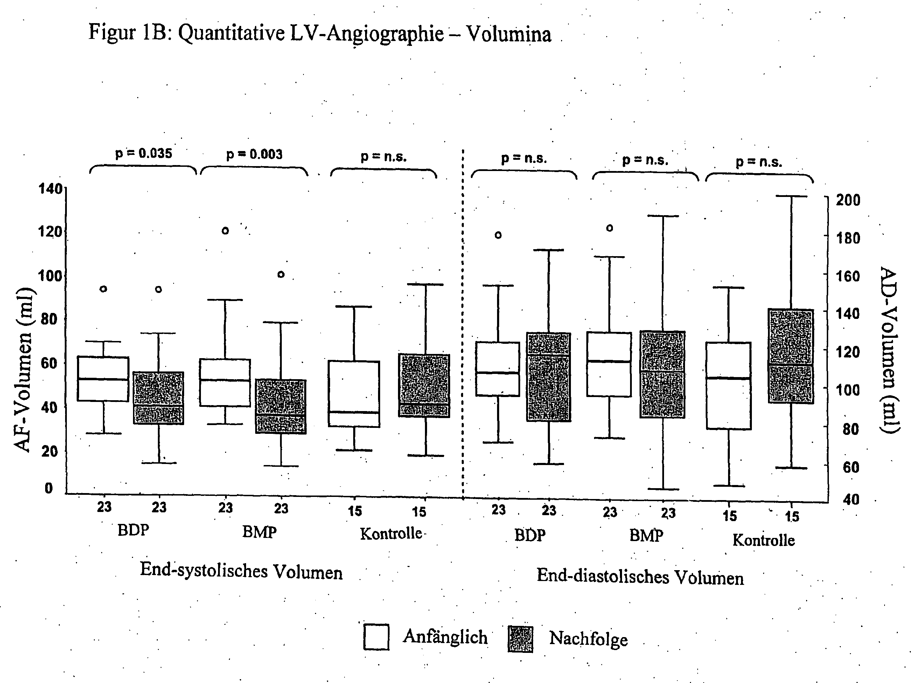

Figur 1 und Tabelle I zeigen die LV-Ausstossfraktion, die End-systolischen und Enddiastolischen LV-Volumina vor und 4 Monate nach intrakoronarer Vorläuferzell-Infusion. Die Transplantation von Vorläuferzellen war mit einer profunden Verbesserung der LV-Ausstossfraktion von 49,2 ± 10 % auf 58,3 ± 10 % (p < 0,001, Figur 1A) und einer signifikanten Verringerung des LV End-systolischen-Volumens von 55,2 ± 18 ml auf 44,1 ± 19 ml (p = 0,009, Figur 1B) assoziiert, irrespektive von dem infundierten Zelltyp. Eine detaillierte Analyse der regionalen Wandbewegung ergab die deutlichsten Verbesserungen im Grenzbereich, der an den zentralen Infarktbereich angrenzte (Tabelle 2).

Im Gegensatz dazu wurde in der Referenzgruppe der Patienten, die keine Vorläuferzell-Infusion erhielten, ansonsten jedoch gleich behandelt wurden, keine signifikanten Veränderungen nachgewiesen (Figur 1 und Tabelle 2). Daher war in der Referenzgruppe, trotz ähnlicher Ausgangswerte, die Verbesserung in der globalen LV-Ausstossfraktion signifikant geringer (p = 0,024) und nahm das End-systolische LV-Volumen nach 4 Monaten eher zu (p = 0,002).

Die Verbesserung der LV-Funktion bei den Vorläuferzell-behandelten Patienten wurde durch Echokardiografie bestätigt und wurde in denjenigen Patienten beibehalten, die zwischenzeitlich einer 12 Monate Nachuntersuchung unterzogen worden (BDP n = 14, BMP n = 10). Keiner dieser Patienten erfuhr irgend eine maligne Arrythmie während der Nachfolgezeit.

Figur zwei zeigt, dass es eine enge inverse Korrelation zwischen Ausgangslinien LV-Ausstossfraktion und Verbesserung in der Ausstossfraktion während der 4 Monate Nachfolge gab, was anzeigte, dass diejenigen Patienten mit der schwersten Beeinträchtigung in der LV-Funktion am meisten im Hinblick auf die Verbesserung der Ausstossfraktion profitierten, unabhängig von dem infundierten Zelltyp.

### Effekte der Vorläufer-Zelltherapie auf die Infarktgrösse, gemessen durch MRI

Die 26 Patienten, die einer seriellen anfänglichen und nach 4 Monaten Nachfolge MRI unterzogen worden und entschieden sich nicht von der gesamten Studie im Hinblick auf die Verbesserung in der LV-Ausstossfraktion (von 50,7 ± 8% auf 60,5 ± 8%, p < 0,001) oder der Verringerung im End-systolischen LV-Volumen (von 53,7 ± 17ml auf 40,8 ± 15ml, p < 0,002). Wichtige Weise war eine intrakoronare Vorläuferzell-Infusion mit einer signifikanten Verringerung im Volumen der Spät-Verstärkung von 39,3 ± 34 auf 31,6 ± 28ml, p < 0,038 vorhanden, was eine signifikante Infarktgrössen-Verringerung anzeigte. Wiederum gab es keine signifikanten Unterschiede zwischen Patienten, die BDP erhielten im Vergleich zu BMP-Patienten.

### Zahl und migratorische Kapazität von transplantierten Vorläuferzellen

Die absolute Zahl der infundierten Vorläuferzellen korrelierte nicht mit einer verbesserten globalen oder regionalen LV-Funktion oder mit der Infarktgrössen-Verringerung, wenn die gesamten Zellzahlen oder Untergruppen (z.B. CD34/CD45 oder CD34/CD133 positive Zellen) verwendet wurden (globale Ausstossfraktion: BDP: r=-0,73, p=0,2; BMP: r=-0,147, p=0,517; Infarktgrösse: BDP: r=-0,187, p=0,5; BMP: r=-0,084, p=0,776). Keine signifikanten Unterschiede wurde bei der funktionellen Verbesserung gefunden, wenn die Zellzahlen dichotomisiert wurden).

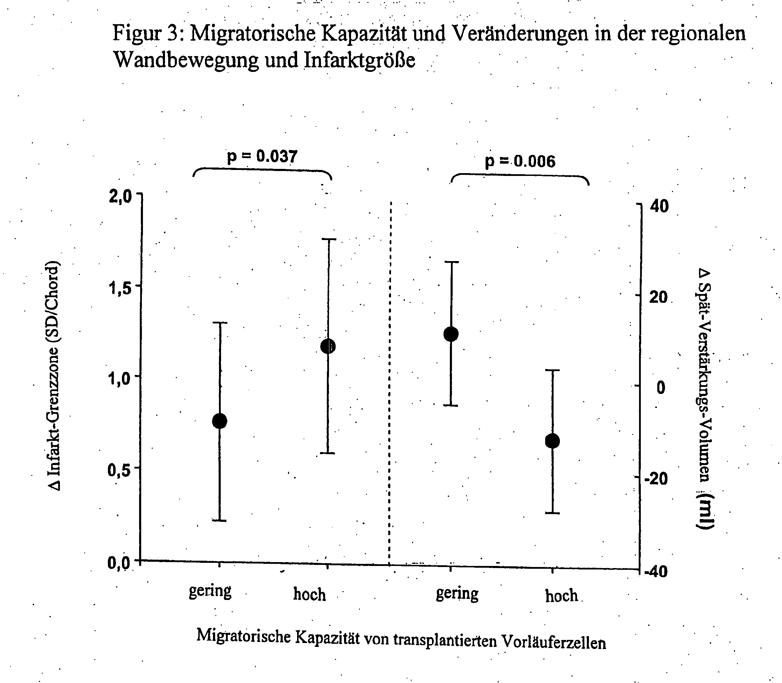

Im Gegensatz dazu ergab die Dichotomisierung der Vorläuferzellen migratorischen Kapazität in diejenigen oberhalb und unterhalb des Mittelwerts, dass Patienten, die Zellen mit einer hohen migratorischen Kapazität erhielten eine signifikante Verbesserung der regionalen LV-Funktion zeigte, wie durch quantitative LV-Angiographie in der Grenzzone des Infarktbereichs gezeigt wurde, verglichen Mitpatienten, die Vorläuferzellen mit geringer migratorischer Kapazität erhielten (Figur 3). Wie in Figur drei dargestellt war die absolute Verringerung in der Spät-Verstärkung Signifikanz höher in Patienten, die Zellen mit einer hohen migratorischen Kapazität erhielten verglichen mit denjenigen, die Vorläuferzellen mit geringer migratorischer Kapazität erhielten.

Jedoch waren weder die klinischen Variablen noch funktionellen Parameter vor der Zelltransplantation, wie z. B. globale LV-Ausstossfraktion, LV End-diastolische und End-systolische Volumina oder die regionale LV-Funktion zwischen beiden Gruppen an der Ausgangslinie verschieden (Tabelle 3), was daher irgend einen potentiellen weiteren Effekte der Schwere der Erkrankung auf die Zell-migratorische Aktivität bei Basislinie ausschloss.

### Multivariante Analyse von unabhängigen Vorhersage-Faktoren der funktionellen Verbesserung und Infarktgrösse-Verringerung

Um unabhängige Vorhersage-Faktoren der funktionellen Verbesserung und Infarktgrösse-Verringerung in Assoziation mit der intrakoronaren Vorläuferzell-Infusion in die Infarktarterie in Patienten mit akutem Myokardinfarkt zu identifizieren, führten die Erfinder einer multivariante Analyse durch, die alle Parameter einschloss, die statistisch signifikant oder näherungsweise durch univariante Analyse statistisch signifikant waren oder von denen bekannt war, dass sie die LV-Funktion oder Infarktgrösse beeinflussen. Wie in Tabelle 4A gezeigt, blieb die migratorische Kapazität der transplantierten Vorläuferzellen der stärkste statistisch signifikante unabhängige Vorhersage-Faktor der Infarktgrössen-Verringerung, wie durch die Verringerung des Spät-Verstärkungsvolumens gemessen wurde. Der einzige andere unabhängige Vorhersage-Faktor war die Basislinien-Ausstossfraktion, wohingegen weder die anfängliche Infarktgrösse, noch das Alter, Geschlecht oder die Zeit bis zur Revaskularisierung unabhängige Vorhersage-Faktoren blieben. Ebenso, wie in Tabelle 4B zusammengefasst, sagte die migratorische Kapazität auch unabhängig die regionale LV-Funktionsverbesserung vorher. Daher ist die migratorische Kapazität der infundierten Zellen eine hauptsächliche unabhängige Determinante der funktionellen Verbesserung und Infarktgrössen-Verringerung im Anschluss an die Vorläuferzellen-Therapie in Patienten mit akutem Myokardinfarkt.

### Zahl und migratorische Kapazität von BMP

Die Charakteristika und funktionelle Aktivität von BMP wurden unter der Verwendung von Knochenmarks Aspiraten von 18 Patienten mit ICMP und 8 gesamten freiwilligen untersucht. Die Basislinien-Charakteristika der Studienpopulationen sind in Tabelle 5 dargestellt.

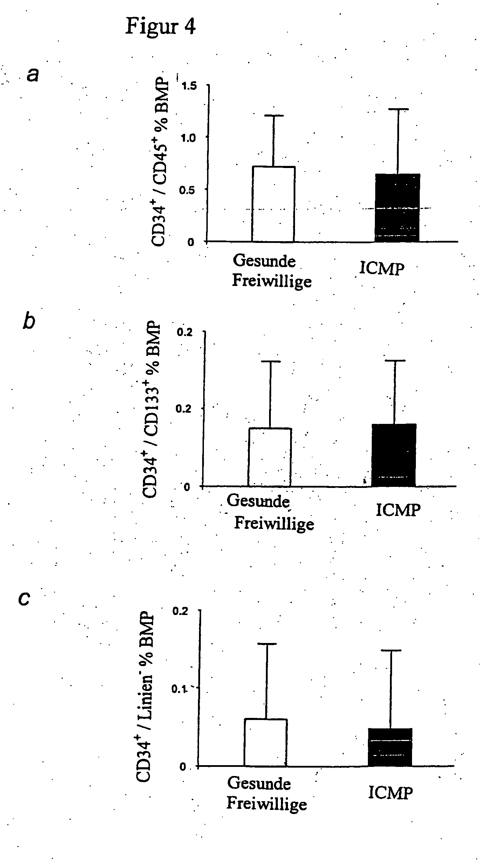

Um zu untersuchen, ob die Zahl von Vorläuferzellen im Hinblick auf BMP bei Patienten mit ICMP im Vergleich zu der gesamten Kontrolle verringert ist, bestimmten von die Zahl von hämatopoietischen Vorläuferzellen, die durch die Expression des Markerproteins CD34 gekennzeichnet ist. Die Zahl von CD34+/CD45+ waren in sowohl der gesunden Kontrolle als auch bei Patienten mit ICMP ähnlich (*Figur 4*)*.* Darüber hinaus unterschieden sich auch die unreiferen Untersets von hämatopoietischen Vorläuferzellen, die als CD133+/CD34+ Zellen und Lineage-/CD34+ definiert wurden, nicht zwischen den Gruppen (Figur 4).

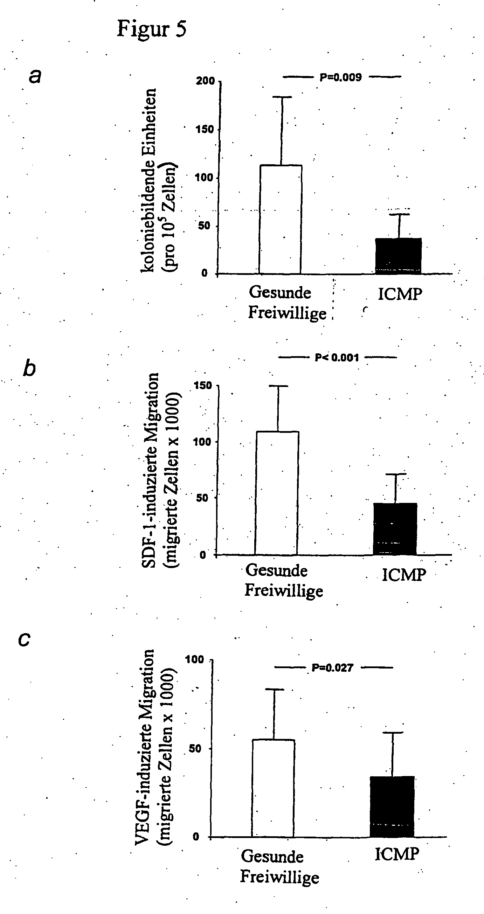

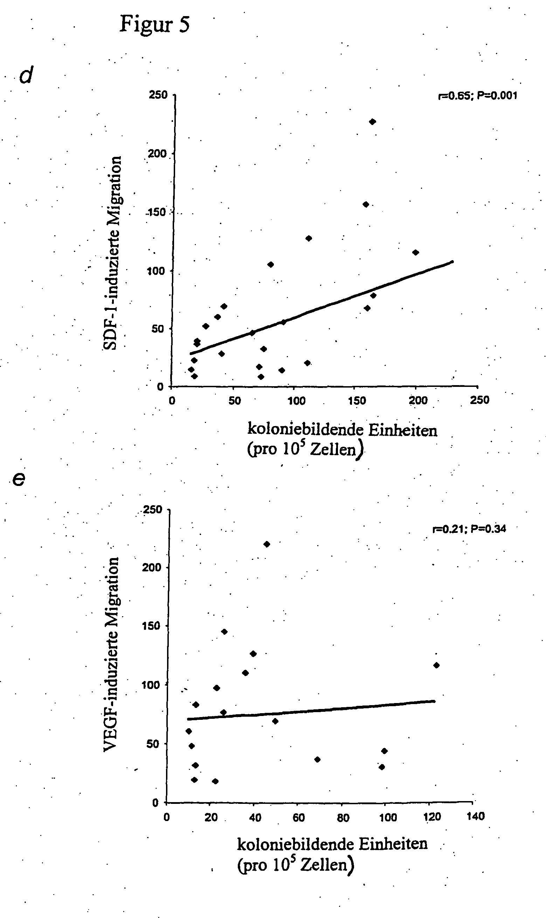

Die Vorläuferzellenaktivität von Vorläuferzellen in Knochenmarks Aspiraten wurde weiterhin durch Messen der koloniebildenden Aktivität und Migration bestimmt. Interessanterweise zeigten BMP von Patienten mit ICMP eine signifikante verringerte Zahl von koloniebildenden Einheiten, verglichen mit BMP aus der gesunden Kontrolle (37,3 ± 25,0 CFU-GM/Schale gegenüber 113,8 ± 70,4 CFU-GM/Schale, p = 0,009) (Figur 5a). Die migratorische Antwort auf SDF-1 und VEGF war auch in den BMP von Patienten mit ICMP Signifikanz verringert, verglichen zu BMP aus der gesunden Kontrolle (VEGF: 34 ± 24,2 gegenüber 54,8 ± 29,3 Zellen/mikroskopisches Feld; p = 0,027; SDF-1: 46,3 ± 26,2 gegenüber 108,6 ± 40,4 Zellen/mikroskopisches Feld; p = 0,001) (Figuren 5b und c) . Übereinstimmender Weise korrigierte die koloniebildende Kapazität eng mit der migratorischen Antwort auf SDF-1 (r = 0,65; p < 0,001) (Figur 5d).

### Unabhängige Vorhersage-Faktoren für die funktionelle Aktivität von BMP

Die SDF-1 induzierte Migration, jedoch nicht die basale Migration oder die VEGF-induzierte Migration korrelierte mit der funktionellen Verbesserung der Neovaskularisierung im Hinterlaufischämie-Modell (r = 0,78; p<0,001) (Figur 6). Die koloniebildende Aktivität der BMP war auch signifikant mit der Neovaskularisierungs-Kapazität der Zellen assoziiert (r = 0,74; p<0,001). Verschiedene klinische Basischarakteristika der Patienten waren auch mit dem therapeutischen Effekt der BMPs in Mausmodell der Hinterlaufischämie assoziiert (Tabelle 6, linke Spalte), jedoch verloren alle Variablen ausser der SDF-1 induzierte Migration ihre statistische Signifikanz, wenn die Analyse auf Patienten mit ICMP begrenzt wurde (Tabelle 6, rechte Spalte).

Für eine Vorhersage eines Schwellenwertes der funktionellen Verbesserung der Neovaskularisierung wurde für eine wie oben beschriebenen Präparation der BMP beispielhaft ein potentieller Schwellenwert bestimmt, der anhand von SDF-1 und VEGF folgende Werte ergab:

**Tabelle A: Migration BMP**

| | | Migration SDF (x 1000) | Migration VEGF (x1000) |
|---|---|---|---|
| N | Gültig | 82 | 83 |
| | Fehlend | 253 | 252 |
| Median | | 70,2500 | 43,0000 |
| Perzentile | | | |
| 25 | | 38,1250 | 22,0000 |
| 33,333 | | 47,6667 | 25,0000 |
| 50 | | 70,2500 | 43,0000 |
| 66,666 | | 104,0000 | 78,2500 |
| 75 | | 120,7500 | 96,0000 |

Die Zahlenwerte geben an, wie viele Zellen der eingesetzten 500.000 Zellen in 24 h transmigriert sind. 100 x1000 bedeutet also das 20 % der Zellen durchgewandert sind.

Für eine Vorhersage eines Schwellenwertes der funktionellen Verbesserung der Neovaskularisierung wurde für eine wie oben beschriebenen Präparation der BDP beispielhaft ein potentieller Schwellenwert bestimmt, der anhand von VEGF folgende Werte ergab:

**Tabelle B: Migration BDP**

| | | Migration VEGF (x1000) |
|---|---|---|
| N | Gültig | 120 |
| | Fehlend | 215 |
| Median | | 4,8500 |
| Perzentile | | |
| 25 | | 2,2250 |
| 33,333 | | 2,8667 |
| 50 | | 4,8500 |
| 66,666 | | 7,7667 |
| 75 | | 10,7000 |

Für BMP wäre ein akzeptabler (bevorzugter) Schwellenwert anhand des Median bei der Untersuchung mit SDF-1 somit 70 x 1000 migrierte Zellen (d.h., ca. 14%). Für BMP wäre ein weiterer akzeptabler (bevorzugter) Schwellenwert anhand des Median bei der Untersuchung mit VEGF somit 43 x 1000 migrierte Zellen (d.h., ca. 8,6%).

Für BDP wäre ein akzeptabler (bevorzugter) Schwellenwert anhand des Median bei der Untersuchung mit VEGF hingegen 4,85 x 1000 migrierte Zellen (d.h., ca. 1 %).

Trotz der Tasache, daß nicht alle Zell-populationen gleich migrieren, kann somit anhand der Gesamtmigration aller Zellen Aufschluss darüber gewonnen werden, wie gut die Zellen des Patienten migrieren und ob diese somit geeignet sind.

**Tabelle 1: Demographische, klinische und angiograpische Charakteristika der Studienpopulation**

| | BDP Gruppe | BMP Gruppe | Kontrolle (nicht randomisiert) | p-Wert |
|---|---|---|---|---|
| | n=23 | n=23 | n= 15 | n.s. |
| Alter (Jahre) | 50 ±10 | 52±10 | 54 ± 13 | n.s. |
| Geschlecht männlich (%) | 87 | 86 | 80 | n.s. |
| Hochdruck (%) | 57 | 48 | 60 | n.s. |
| Hyperlipidämie (%) | 7 | 60 | 80 | n.s. |
| Diabetes (%) | 17 | 27 | 7 | n.s. |
| Raucher (%) | 83 | 91 | 73 | n.s. |
| Packjahre | 32 ± 24 | 35 ±23 | n.b. | n.s. |
| Familienhistorie auf KHE (%) | 26 | 50 | 40 | n.s. |
| KHE (1/2/3 Ge-fässerkrankung %) | 74/26/0 | 59/41/0 | 9/3/3 | n.s. |
| Historie von KHE (%) | 0 | 0 | 0 | n.s. |
| Infarktbereich (anterior / inferi-or) (%) | 65/35 | 50/50 | 60/40 | n.s. |
| Infarkt zusam-menhängendes Gefäss (%) | | | | |
| LAD | 61 | 46 | 53 | n.s. |
| LCX | 22 | 4 | 13 | n.s. |
| RCA | 17 | 5 | 34 | n.s. |
| Primäre Therapie | 91 | 73 | 93 | n.s. |
| PTCA / Stent (n) Zeit zur Neo-vaskularisierung (Mittelwert / Median) | 17 ±27/4 | 32 ±49/14 | 16 ±24/5 | n.s. |
| TIMI III Fluss post Reperfusion (%) | 87 | 91 | n.b. | n.s. |
| Ausstossfraktion (visuell ge-schätzt) | 43 ± 11 | 40 ± 8 | 40 ± 7 | n.s. |
| CPR während akuter MI (n) | 0 | 2 | 1 | n.s. |
| Kreatinkinase max. (U/1)* | 940 ±725 | 781 ±630 | 915 ± 740 | n.s. |
| CK MB max. (U/1)* | 104 ± 67 | 94±106 | 146±128 | n.s. |
| Medikation nach Entlassung Aspirin (%) | 100 | 100 | 93 | n.s. |
| Clopidogel (%) | 100 | 100 | 100 | n.s. |
| ACE-Inhibitor (%) | 96 | 100 | 93 | n.s. |
| Betablocker (%) | 100 | 100 | 100 | n.s. |
| Statin (%) | 100 | 100 | 93 | n.s. |
| Zeit Stent zur Zelltherapie (h) | 119±27 | 111±41 | - | n.s. |
| Zahl von injizier-ten Zellen (Mio.) | 17 ± 12 | 219 ± 82 | - | < 0,001 |
| **Vor Zellthera-pie** | | | | |
| Zahl der weissen Blutzellen (/nl) | 12,4 ± 4,2 | 12,8 ± 3,6 | - | n.s. |
| C-reaktives Pro-tein (mg/dl) | 2,8 ± 2,2 | 3,2 ± 2,4 | - | n.s. |
| Troponin T (ng/ml) | 2,0 ±1,4 | 2,2 ±1,9 | - | |
| **24 Stunden nach Zellthera-pie** | | | | |
| Zahl der weissen Blutzellen (/nl) | 8,5 ±2,1 | 9,8 ±1,9 | - | 0,02 |
| C-reaktives Pro-tein (mg/dl) | 2,6 ± 2,3 | 3,1 ± 1,9 | - | n.s. |
| Troponin T (ng/ml) | 1,3 ± 1,0 | 1,5 ±1,2 | - | n.s. |

| | | | | |
|---|---|---|---|---|
| CPR = Kardio-pulmonale Wiederbelebung *ohne CPR-Patienten | | | | |

**Tabelle 2: Quantitative globale und regionale LV Funktion**

| BDP (n = 23) | Basislinie | Nachfolge | p-Wert |
|---|---|---|---|
| Ausstoss-Fraktion (%) | 49 ± 10 | 58 ±10 | 0,004 |
| End-diastolisches Volumen (ml) | 107 ±25 | 108 ± 33 | n.s. |
| End-systolisches Vo- lumen (ml) | 53 ± 14 | 45 ± 18 | 0,035 |
| Regionale Wandbe- wegung (SD/Chord) | | | |
| Infarkt | -1,5 ±0,3 | -0,7 ±0,6 | < 0,001 |
| Infarktmitte | -1,6 ± 0,4 | - 0,8 ±0,7 | < 0,001 |
| Infarktgrenze | - 1,5 ± 0,2 | - 0,5 ± 0,6 | < 0,001 |

| BMP (n = 23) | Basislinie | Nachfolge | p-Wert |
|---|---|---|---|
| Ausstoss-Fraktion (%) | 49 ±10 | 59 ±9 | 0,003 |
| End-diastolisches Volumen (ml) | 114 ±29 | 101 ± 33 | n.s. |
| End-systolisches Vo-lumen (ml) | 57 ± 21 | 43 ± 21 | 0,003 |
| Regionale Wandbe-wegung (SD/Chord) | | | |
| Infarkt | -1,5 ± 0,3 | - 0,5 ± 0,6 | < 0,001 |
| Infarktmitte | - 1,7 ± 0,4 | - 0,7 ± 0,6 | < 0,001 |
| Infarktgrenze | -1,4 ±0,2 | - 0,4 ± 0,7 | < 0,001 |
| | | | |

| Kontrolle (n = 15) | Basislinie | Nachfolge | p-Wert |
|---|---|---|---|
| Ausstoss-Fraktion (%) | 50,3 ±9,3 | 52,6 ±9,4 | 0,379 |
| End-diastolisches Volumen (ml) | 101,3 ± 22,6 | 120,0 ±47,0 | 0,108 |
| End-systolisches Vo-lumen (ml) | 50,9 ±16,7 | 57,6 ± 30,6 | 0,363 |
| Regionale Wandbe-wegung (SD/Chord) | n.d. | n.d. | - |

**Tabelle 3: Ausgangslinien Parameter zwischen Patienten, die Zellen mit einer hohen migratorischen Kapazität erhielten verglichen mit denjenigen, die Vorläuferzellen mit geringer migratorischer Kapazität erhielten**

| Basislinie | hohe migratorische Kapazität n=16 | geringe migratorische Kapazität n = 15 | p-Wert |
|---|---|---|---|
| Alter (Jahre) | 48 ± 11 | 50 ± 9 | n.s. |
| Geschlecht (männl./weibl.) | 15/1 | 13/2 | n.s. |
| Zahl Risikofaktoren (%) | 2,7 ± 0,9 | 2,6 ± 0,9 | n.s. |
| Infarktgebiet (ant./inf.; %) | 75/25 | 60/40 | n.s. |
| Ausstoss-Fraktion (%) | 48 ± 8 | 49 ± 12 | n.s. |
| End-diastolisches Volumen (ml) | 104 ±22 | 118 ± 25 | n.s. |
| End-systolisches Vo-lumen (ml) | 53 ± 15 | 59 ±20 | n.s. |
| Regionale Wandbe-wegung (SD/Chord) | | | n.s. |
| Infarkt | -1,5 ±0,2 | -1,5 ± 0,3 | n.s. |
| Infarktmitte | -1,7 ± 0,3 | - 1,6 ± 0,3 | n.s. |
| Infarktgrenze | - 1,4 ± 0,2 | - 1,5 ±0,3 | n.s. |
| Trennwand Bewe-gungswert | 7,0 ± 2,4 | 6,7 ±3,0 | n.s. |
| Spät-Verstärkungsvolumen (ml) | 39 ± 44 | 35 ± 22 | n.s. |

**Tabelle 4A: Multivariante Analyse der unabhängigen Vorhersage-Faktoren der Infarktgrössen-Verringerung**

| | Δ Spät-Verstärkungsvolumen r | p-Wert |
|---|---|---|
| Geschlecht | - 0,094 | n.s. |
| Alter | - 0,099 | n.s. |
| Zeit zur Revaskularisierung | 0,329 | n.s. |
| Basislinien Ausstossfraktion | 0,784 | 0,016 |
| Basislinien Spät-Verstärkungsvolumen | 0,184 | n.s. |
| Hohe / geringe Migration r = Korrelationskoeffizient | - 0,883 | 0,007 |

**Tabelle 4B: Multivariante Analyse der unabhängigen Vorhersage-Faktoren der regionalen Verbesserung der LV-Funktion**

| | Δ Trennwand-Bewegungswert r | p-Wert |
|---|---|---|
| Geschlecht | 0,076 | n.s. |
| Alter | 0,6 | 0,035 |
| Zeit zur Revaskularisierung | 0,165 | n.s. |
| Basislinien Ausstossfraktion | 0,403 | n.s. |
| Basislinien Trennwand- Bewegungswert | - 0,735 | 0,008 |
| Hohe / geringe Migration | - 0,619 | 0,021 |

**Tabelle 5: Basislinien-Charakteristika der Studienpopulation**

| | Patienten mit ICMP | Gesunde Kon trolle | p-Wert |
|---|---|---|---|
| | n= 18 | n=8 | |
| Alter (Jahre) | 59,4 ±13,4 | 54 ±13 | < 0,001 |
| Geschlecht männlich (%) | 88,9 | 87,5 | 0,65 |
| Hochdruck (%) | 57 | 0 | < 0,001 |
| Hyperlipidämie (%) | 70 | 25 | < 0,001 |
| Diabetes (%) | 17 | 0 | < 0,001 |
| Raucher (%) | 82,4 | 37,5 | < 0,001 |
| Familienhistorie auf KHE (%) | 52,9 | 25,0 | < 0,001 |
| KHE (1/2/3 Gefässerkrankung %) | 17,6/52,9/29,4 | n.a. | |
| Historie von KHE (%) | 100 | n.a. | |
| Infarktbereich (anterior / inferior)(%) | 65/35 | n.a. | |
| Ausstossfraktion (visuell geschätzt) | 37,8 ± 11,1 | 62,6 ± 3,1 | < 0,001 |
| Medikation nach Entlassung | | | |
| Aspirin (%) | 100 | 0 | |
| Clopidogel (%) | 100 | 0 | |
| ACE-Inhibitor (%) | 100 | 0 | |
| Betablocker (%) | 100 | 0 | |
| Statin (%) | 100 | 0 | |
| C-reaktives Protein (mg/dl) | 3,0 ± 2,5 | 2,9 ± 1,7 | 0,51 |
| Zahl der weissen Blutzellen (x 10³/nl) | 7,9 ± 3,7 | 6,8 ± 2,9 | 0,85 |

**Tabelle 6: Univariate lineare Regressionsanalyse des Zusammenhangs zwischen klinischen Charakteristika sowie den Messungen in vitro und der Neovaskularisierungs-Kapazität von BMP in einem Mausmodell von Hinterlaufischämie**

| | Patienten und gesunde Kontrollen | | Nur Patienten | |
|---|---|---|---|---|
| | r | p-Wert | r | p-Wert |
| Alter | - 0,38 | 0,064 | 0,260 | 0,33 |
| Geschlecht | 0,032 | 0,87 | - 0,165 | 0,49 |
| Hochdruck | - 0,646 | **0,001** | - 0,204 | 0,43 |
| Hyperlipidämie | - 0,598 | **0,002** | 0,114 | 0,68 |
| Familienhistorie | -0,403 | **0,019** | 0,134 | 0,61 |
| auf KHE | | | | |
| Diabetes | - 0,641 | **0,001** | - 0,088 | 0,74 |
| Zahl der Risiko faktoren | - 0,540 | **0,006** | 0,174 | 0,52 |
| NYHA Klassifizierung | - 0,630 | **0,001** | -0,116 | 0,66 |
| Ausstossfraktion (%) | 0,720 | **0,001** | 0,315 | 0,273 |
| Basale Migration | 0,380 | 0,061 | 0,456 | **0,05** |
| SDF-1 induzierte Migration | 0,781 | <**0,001** | 0,634 | **0,004** |
| VEGF induzierte Migration | 0,352 | 0,078 | 0,374 | 0,13 |
| Koloniebildende Kapazität | 0,740 | <**0,001** | 0,074 | 0,785 |
| r = Korrelationskoeffizient | | | | |

## Patentansprüche

1. *In vitro* Verfahren zur Stratifizierung von Patienten mit kardiovaskulären Erkrankungen für eine geplante Zelltherapie auf Basis von Knochenmarks-Vorläuferzellen (BMP) und/oder Blut-abgeleiteter zirkulierender Vorläuferzellen (BDP) zur Steigerung der Perfusion von ischämischem Gewebe bzw. zur Regeneration von Gewebeverlust, wobei das Verfahren folgende Schritte umfaßt:
a) Isolieren von BMP und/oder BDP mittels zellspezifischer Oberflächenmarker aus einer Probe, und
b) Überprüfung der kardiovaskulären Funktionalität der isolierten BMP und/oder BDP mittels eines geeigneten Migrationstests.

2. Verfahren nach Anspruch 1, weiterhin umfassend den Vergleich des aus der untersuchten Probe erhaltenen Ergebnisses mit einem Referenzwert und/oder dem Ergebnis einer Referenzprobe.

3. Verfahren nach Anspruch 1 oder 2, wobei die zu untersuchende Probe ausgewählt ist aus der Gruppe bestehend aus Knochenmark, peripherem Blut oder Fraktionen davon und Zellkultur-Suspensionen oder Fraktionen davon.

4. Verfahren nach Anspruch 3, wobei die zu untersuchende Probe mittels Punktion aus dem Knochenmark entnommen wurde.

5. Verfahren nach Anspruch 3, wobei dem peripheren Blut ein Gerinnungshemmer zugesetzt wird.

6. Verfahren nach einem der Ansprüche 1 bis 5, wobei die Isolierung die Verwendung von Dichte-Gradienten-Zentrifugation und/oder immunologischer Methoden umfaßt.

7. Verfahren nach einem der Ansprüche 1 bis 6, wobei der zellspezifische Oberflächenmarker für BMP ausgewählt ist aus CD34, CD45 und/oder CD133, und für BDP ausgewählt ist aus VEGFR2, CD105, vWF und/oder CD31.

8. Verfahren nach einem der Ansprüche 1 bis 7, wobei der Migrationstest in einer Boyden-Kammer oder modifizierten Boyden-Kammer durchgeführt wird.

9. Verfahren nach einem der Ansprüche 1 bis 8, wobei der Migrationstest unter der Verwendung von SDF-1 für BMP oder VEGF für BDP oder P1GF oder MCP-1 durchgeführt wird.

10. Verfahren nach einem der Ansprüche 1 bis 9, wobei die kardiovaskulären Erkrankungen ausgewählt sind aus der Gruppe bestehend aus stabiler und instabiler Angina, stabiler koronarer Herzerkrankung, akutem Koronarsyndrom, Myokardinfarkt, akutem Myokardinfarkt, akutem Herzsyndrom, koronarer Arterienerkrankung, chronischer ischämischer Kardiomyopathie (ICMP), dilatativer Kardiomyopathie (DCM), Herzinsuffizienz oder anderen Ursachen einer Herzschwäche.

11. Verfahren nach einem der Ansprüche 1 bis 10, wobei die untersuchten isolierten BMP und/oder BDP für den Patienten autolog sind.

12. *In vitro* Verfahren zur Überprüfung der kardiovaskulären Funktionalität von spezifischen Knochenmarks-Vorläuferzellen (BMP) und/oder Blut-abgeleiteter zirkulierender Vorläuferzellen (BDP) aus einer Probe aus einem Säugetier-Spender, umfassend:
a) zur Verfügung stellen einer Probe und
b) Durchführen eines Verfahrens nach einem der Ansprüche 1 bis 11.

13. Verfahren nach Anspruch 12, wobei die isolierten BMP und/oder BDP weiter genetisch modifiziert werden.

## Claims

1. *In vitro* method for a stratification of patients with cardiovascular diseases for a scheduled cell therapy on the basis of bone marrow precursor cells (BMP) and/or blood-derived circulating precursor cells (BDP) for an increase of the perfusion of ischemic tissue or for the regeneration of tissue loss, respectively, wherein the method comprises the following steps:
a) isolating of BMP and/or BDP by means of cell specific surface markers from a sample, and
b) determining of the cardiovascular functionality of the isolated BMP and/or BDP by means of a suitable migration assay.

2. Method according to claim 1, further comprising the comparison of the result as obtained from the sample as examined with a reference value and/or the result of a reference sample.

3. Method according to claim 1 or 2, wherein the sample to be examined is selected from the group consisting of bone marrow, peripheral blood or fractions thereof, and cell culture-suspensions or fractions thereof.

4. Method according to claim 3, wherein the sample to be examined is obtained by means of punctuation from the bone marrow.

5. Method according to claim 3, wherein a coagulation inhibitor is added to the peripheral blood.

6. Method according to any of claims 1 to 5, wherein isolating comprises using density-gradient-centrifugation, cell specific surface markers, and/or immunological methods.

7. Method according to any of claims 1 to 6, wherein the cell specific surface marker for BMP is selected from CD34, CD45, and/or CD133, and for BDP is selected from VEGFR2, CD105, vWF, and/or CD31.

8. Method according to any of claims 1 to 7, wherein the migration assay is performed in a Boyden-chamber or a modified Boyden-chamber.

9. Method according to any of claims 1 to 8, wherein the migration assay is performed using SDF-1 for BMP or VEGF for BDP, or P1GF or MCP-1.

10. Method according to any of claims 1 to 9, wherein the cardiovascular diseases are selected from the group consisting of stable and unstable angina, stable coronary heart disease, acute coronary syndrome, myocardial infarction, acute myocardial infarction, acute heart syndrome, coronary artery disease, chronic ischemic cardiomyopathy (ICMP), dilatative cardiomyopathy (DCM), heart insufficiency, or other causes of a cardiac weakness.

11. Method according to any of claims 1 to 10, wherein the studied isolated BMPs and/or BDPs are autologous for the patient.

12. *In vitro* method for determining of the cardiovascular functionality of specific bone marrow-precursor-cells (BMP) and/or blood-derived circulating precursor-cells (BDP) from a sample from a donor-mammal, comprising:
a) providing of a sample, and
b) performing a method according to any of claims 1 to 11.

13. Method according to claim 12, wherein the isolated BMP and/or BDP are further genetically modified.

## Revendications

1. Procédé *in vitro* pour stratifier des patients atteints de pathologies cardio-vasculaires en vue d'une thérapie cellulaire à base de cellules souches de la moelle osseuse (CSMO) et/ou de cellules souches circulantes d'origine sanguine (CSCS) destinée à accroître la perfusion de tissu ischémique ou à régénérer une perte de tissu, ledit procédé comprenant les étapes consistant à :
a) isoler des CSMO et/ou des CSCS à partir d'un échantillon en utilisant des marqueurs de surface spécifiques aux cellules, et
b) contrôler la fonctionnalité cardiovasculaire des CSMO et/ou CSCS isolées au moyen d'un essai de migration approprié.

2. Procédé selon la revendication 1, comprenant en plus la comparaison du résultat obtenu avec l'échantillon analysé avec une valeur de référence et/ou avec le résultat obtenu avec un échantillon de référence.

3. Procédé selon la revendication 1 ou la revendication 2, dans lequel l'échantillon à analyser est choisi dans le groupe constitué par de la moelle osseuse, du sang périphérique ou des fractions de celui-ci, et des suspensions de culture cellulaire ou des fractions de celles-ci.

4. Procédé selon la revendication 3, dans lequel l'échantillon à analyser a été prélevé par une ponction de moelle osseuse.

5. Procédé selon la revendication 3, dans lequel le sang périphérique est additionné d'un inhibiteur de coagulation.

6. Procédé selon l'une des revendications 1 à 5, dans lequel l'étape d'isolement comprend l'utilisation de la centrifugation en gradient de densité et/ou de méthodes immunologiques.

7. Procédé selon l'une des revendications 1 à 6, dans lequel le marqueur de surface spécifique aux cellules est choisi pour les CSMO parmi le CD34, le CD45 et/ou le CD133, et pour les CSCS parmi le VEGFR2, le CD105, le vWF et/ou le CD31.

8. Procédé selon l'une des revendications 1 à 7, dans lequel l'essai de migration s'effectue dans une chambre de Boyden ou dans une chambre de Boyden modifiée.

9. Procédé selon l'une des revendications 1 à 8, dans lequel l'essai de migration s'effectue en utilisant le SDF-1 pour les CSMO ou le VEGF pour les CSCS, ou bien le P1GF ou le MCP-1.

10. Procédé selon l'une des revendications 1 à 9, dans lequel les pathologies cardiovasculaires sont choisies dans le groupe constitué par l'angor stable et instable, la maladie coronaire stable, le syndrome coronaire aigu, l'infarctus du myocarde, l'infarctus du myocarde aigu, le syndrome cardiaque aigu, la maladie des artères coronaires, la cardiopathie ischémique chronique (CIC), la cardiomyopathie dilatée (CMD), l'insuffisance cardiaque ou d'autres causes de faiblesse cardiaque.

11. Procédé selon l'une des revendications 1 à 10, dans lequel les CSMO et/ou CSCS isolées analysées sont autologues pour les patients.

12. Procédé *in vitro* pour contrôler la fonctionnalité cardiovasculaire de cellules souches de la moelle osseuse (CSMO) et/ou de cellules souches circulantes d'origine sanguine (CSCS) spécifiques à partir d'un échantillon provenant d'un donneur mammifère, comprenant les étapes consistant à :
a) obtenir un échantillon, et
b) mettre en oeuvre un procédé selon l'une des revendications 1 à 11.

13. Procédé selon la revendication 12, dans lequel les CSMO et/ou CSCS isolées sont également soumises à une modification génétique.
